# EUROPÄISCHE PATENTSCHRIFT

(11) **EP 2 373 632 B1**
(45) Veröffentlichungstag und Bekanntmachung des Hinweises auf die Patenterteilung: **04.03.2015**
(21) Anmeldenummer: 09756686.3
(22) Anmeldetag: 26.11.2009
(51) Int. Cl.: C07D 249/12, A61K 31/4196, A61P 9/00

(54) **SUBSTITUIERTE BENZYL- UND PHENYLSULFONYLTRIAZOLONE UND IHRE VERWENDUNG**
SUBSTITUTED BENZYL AND PHENYLSULFONYL TRIAZOLONES, AND USE THEREOF
BENZYLTRIAZOLONES ET PHÉNYLSULFONYLTRIAZOLONES SUBSTITUÉES ET LEUR UTILISATION

(30) Priorität: 06.12.2008 DE 102008060967
(43) Veröffentlichungstag der Anmeldung: 12.10.2011
(73) Patentinhaber: Bayer Intellectual Property GmbH, 40789 Monheim (DE)
(72) Erfinder: BRÜGGEMEIER, UIf, 42799 Leichlingen (DE); FLAMME, Ingo, 51580 Reichshof (DE); FÜRSTNER, Chantal, 45478 Mülheim (DE); KELDENICH, Joerg, 42113 Wuppertal (DE); KOLKHOF, Peter, 42113 Wuppertal (DE); DELBECK, Martina, 45239 Essen (DE); KRETSCHMER, Axel, 42113 Wuppertal (DE); POOK, Elisabeth, 42109 Wuppertal (DE); SCHMECK, Carsten, 45472 Mülheim (DE); TRUEBEL, Hubert, 42281 Wuppertal (DE)
(74) Vertreter: BIP Patents
(86) Internationale Anmeldenummer: PCT/EP2009/008418
(87) Internationale Veröffentlichungsnummer: WO 2010/063402

(56) Entgegenhaltungen:
- EP-A1- 0 636 614
- EP-A2- 0 412 594
- WO-A1-2007/134862
- DE-A1- 19 929 348
- US-B1- 6 746 989
- US-B1- 6 762 152
- US-B1- 6 838 415

## Beschreibung

Die vorliegende Anmeldung betrifft neue, substituierte Benzyl-1,2,4-triazolone und Phenylsulfonyl-1,2,4-triazolone, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von kardiovaskulären Erkrankungen.

Der Flüssigkeitsgehalt des menschlichen Körpers unterliegt verschiedenen physiologischen Kontrollmechanismen, die auf seine Konstanterhaltung abzielen (Volumenhomöostase). Dabei werden sowohl die Volumenfüllung des Blutgefäßsystems als auch die Osmolarität des Blutplasmas kontinuierlich von entsprechenden Sensoren (Barorezeptoren und Osmorezeptoren) registriert. Die Informationen, die diese Sensoren an die zuständigen Zentren des Hirns liefern, regulieren das Trinkverhalten und steuern vermittels humoraler und nervaler Signale die Flüssigkeitsausscheidung über die Nieren. Eine zentrale Bedeutung kommt hierbei dem Peptidhormon Vasopressin zu [Schrier R.W., Abraham, W.T., New Engl. J. Med. 341, 577-585 (1999)].

Vasopressin wird in spezialisierten, endokrinen Neuronen im *Nucleus supraopticus* und *N. paraventricularis* in der Wand des dritten Ventrikels (Hypothalamus) produziert und von dort entlang ihrer Nervenfortsätze in den Hypophysenhinterlappen (Neurohypophyse) verbracht. Dort wird das Hormon je nach Stimulus in die Blutbahn abgegeben. Ein Volumenverlust, z.B. infolge akuter Blutung, starkem Schwitzen, langem Dursten oder Diarrhöe, ist ein Stimulus für die verstärkte Ausschüttung des Hormons. Umgekehrt wird die Sekretion von Vasopressin durch eine Erhöhung des Intravasalvolumens, z.B. infolge gesteigerter Flüssigkeitszufuhr, gehemmt.

Vasopressin entfaltet seine Wirkung hauptsächlich über Bindung an drei Rezeptoren, die als V1a-, V1b- und V2-Rezeptoren klassifiziert werden und zur Familie der G-Protein-gekoppelten Rezeptoren gehören. V1a-Rezeptoren sind vorwiegend auf den Zellen der glatten Gefäßmuskulatur lokalisiert. Ihre Aktivierung ruft eine Vasokonstriktion hervor, wodurch der periphere Widerstand und Blutdruck ansteigen. Daneben sind V1a-Rezeptoren auch in der Leber nachweisbar. V1b-Rezeptoren (auch V3-Rezeptoren genannt) sind im zentralen Nervensystem nachweisbar. Zusammen mit dem Corticotropin-Releasing-Hormon (CRH) reguliert Vasopressin über den Vlb-Rezeptor die basale und stressinduzierte Sekretion des Adrenocorticotropen Hormons (ACTH). V2-Rezeptoren finden sich im distalen Tubulusepithel und dem Epithel der Sammelrohre der Niere. Ihre Aktivierung macht diese Epithelien für Wasser durchlässig. Diesem Phänomen liegt der Einbau von Aquaporinen (speziellen Wasserkanälen) in die luminale Membran der Epithelzellen zugrunde.

Welche Bedeutung Vasopressin für die Rückresorption von Wasser aus dem Harn in der Niere hat, wird durch das Krankheitsbild des Diabetes insipidus deutlich, das durch einen Mangel des Hormons - z.B. infolge einer Hypophysenschädigung - hervorgerufen wird. Patienten, die an diesem Krankheitsbild leiden, scheiden bis zu 20 Liter Harn pro 24 Stunden aus, sofern ihnen das Hormon nicht substituiert wird. Dieses Volumen entspricht in etwa 10% des Primärhams. Wegen seiner großen Bedeutung für die Rückresorption von Wasser aus dem Harn wird Vasopressin auch synonym als Antidiuretisches Hormon (ADH) bezeichnet. Folgerichtig führt eine pharmakologische Hemmung der Vasopressin/ADH-Wirkung am V2-Rezeptor zu einer vermehrten Urinausscheidung. Im Gegensatz zu der Wirkung anderer Diuretika (Thiazide und Schleifendiuretika) jedoch bewirken V2-Rezeptor-Antagonisten eine vermehrte Wasserausscheidung, ohne die Ausscheidung von Elektrolyten maßgeblich zu steigern. Das bedeutet, dass durch V2-antagonistische Pharmaka die Volumenhomöostase wiederhergestellt werden kann, ohne dabei in die Elektrolyt-Homöostase einzugreifen. Daher erscheinen V2-antagonistisch wirksame Pharmaka besonders geeignet für die Behandlung aller krankhaften Zustände, die mit einer Überfrachtung des Organismus mit Wasser einhergehen, ohne dass parallel die Elektrolyte adäquat erhöht sind. Eine bedeutende Elektrolyt-Abnormalität ist klinisch-chemisch als Hyponatriämie (Natriumkonzentration < 135 mmol/L) messbar; sie stellt die wichtigste Elektrolyt-Abnormalität bei Krankenhauspatienten dar mit einer Häufigkeit von ca. 5% bzw. 250.000 Fällen pro Jahr allein in den USA. Bei einem Absinken der Plasma-Natriumkonzentration unter 115 mmol/L drohen komatöse Zustände und Tod.

Entsprechend der zugrunde liegenden Ursache unterscheidet man die hypovolämische, euvolämische und hypervolämische Hyponatriämie. Klinisch bedeutsam sind die Formen der Hypervolämie mit Ödembildung. Typische Beispiele hierfür sind das Syndrom der inadäquaten ADH/Vasopressin-Sekretion (SIAD) (z.B. nach Schädel-Hirn-Trauma oder als Paraneoplasie bei Carcinomen) und die hypervolämische Hyponatriämie bei Leberzirrhose, verschiedenen Nierenerkrankungen und Herzinsuffizienz [De Luca L. et al., Am. J. Cardiol. 96 (suppl.), 19L-23L (2005)]. Gerade Patienten mit Herzinsuffizienz weisen trotz ihrer relativen Hyponatriämie und Hypervolämie häufig erhöhte Vasopressin-Spiegel auf, was als die Folge einer generell gestörten neurohumoralen Regulation bei der Herzinsuffizienz angesehen wird [Francis G. S. et al., Circulation 82, 1724-1729 (1990)].

Die gestörte neurohumorale Regulation manifestiert sich im Wesentlichen in einer Erhöhung des Sympathicotonus und einer inadäquaten Aktivierung des Renin-Angiotensin-Aldosteron-Systems. Während die Hemmung dieser Komponenten durch Beta-Rezeptoren-Blocker einerseits und durch ACE-Inhibitoren bzw. Angiotensin-Rezeptor-Blocker andererseits heute fester Bestandteil der pharmakologischen Behandlung der Herzinsuffizienz ist, ist die inadäquate Steigerung der Vasopressin-Sekretion in der fortgeschrittenen Herzinsuffizienz derzeit noch nicht ausreichend therapierbar. Neben der durch V2-Rezeptoren vermittelten Retention von Wasser und den damit verbundenen ungünstigen hämodynamischen Folgen im Sinne einer Nachlasterhöhung werden auch durch V1a-vermittelte Vasokonstriktion die Entleerung des linken Ventrikels, der Druck in den Pulmonalgefäßen und die Herzleistung negativ beeinflusst. Darüber hinaus wird aufgrund tierexperimenteller Daten dem Vasopressin auch eine direkte Hypertrophie-fördernde Wirkung am Herzmuskel beigemessen. Im Unterschied zur renalen Wirkung der Volumenexpansion, die über Aktivierung von V2-Rezeptoren vermittelt ist, wird die direkte Wirkung am Herzmuskel durch Aktivierung von V1a-Rezeptoren ausgelöst.

Aus diesen Gründen erscheinen Substanzen, die die Wirkung des Vasopressins am V2- und/oder am V1a-Rezeptor hemmen, zur Behandlung der Herzinsuffizienz geeignet. Vor allem Verbindungen mit kombinierter Aktivität an beiden Vasopressin-Rezeptoren (V1a und V2) sollten sowohl wünschenswerte renale als auch hämodynamische Effekte bewirken und somit ein besonders ideales Profil für die Behandlung von Patienten mit Herzinsuffizienz bieten. Die Bereitstellung derartig kombinierter Vasopressin-Antagonisten erscheint auch insofern sinnvoll, als ein allein über V2-Rezeptor-Blockade vermittelter Volumenentzug die Erregung von Osmorezeptoren und in der Folge eine weitere kompensatorische Steigerung der Vasopressin-Ausschüttung nach sich ziehen kann. Hierdurch könnten - bei Fehlen einer gleichzeitig den V1a-Rezeptor blockierenden Komponente - die schädlichen Wirkungen des Vasopressins, wie z.B. Vasokonstriktion und Herzmuskelhypertrophie, noch verstärkt werden [Saghi P. et al., Europ. Heart J. 26, 538-543 (2005)].

In WO 99/31099 werden Sulfonyltriazolone und Triazolonalkylcarbonsäure-Derivate als Integrin Antagonisten beispielsweise zur Behandlung von Tumorerkrankungen offenbart. 5-Aryl-1,2,4-triazolone als Vasopressin-Antagonisten werden in WO 2007/134862 beschreiben. EP 0 533 276-A1 beschreibt unter anderem Alkoxy-substituierte Triazolone als Herbizide. In WO 99/54315 werden Triazolone mit neureprotektiver Wirkung beschrieben. WO 2006/066133 beansprucht substituierte Aminoimidazolinone als HDAC-Inhibitoren zur Behandlung von Krebs- und Entzündungserkrankungen.

Aufgabe der vorliegenden Erfindung ist die Bereitstellung neuer Verbindungen, die als potente selektive V2- und um duale V1a/V2-Rezeptor-Antagonisten wirken und und als solche zur Behandlung und/oder Prävention von Krankheiten, insbesondere zur Behandlung und/oder Prävention von kardiovaskulären Erkrankungen geeignet sind.

Gegenstand der vorliegenden Erfindung sind Verbindungen der allgemeinen Formel (I) 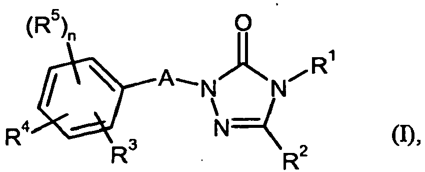 in welcher
- A: für -CH₂- oder -SO₂- steht,
- R¹: für Wasserstoff, (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Halogen, Cyano, Oxo, Trifluormethyl, (C₃-C₇)-Cycloalkyl, Phenyl, -OR¹⁰, -NR¹¹R¹², -C(=O)-OR¹³ und -C(=O)-NR¹⁴R¹⁵ substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹², R¹³, R¹⁴ und R¹⁵ unabhängig voneinander jeweils für Wasserstoff oder (C₁-C₆)-Alkyl stehen,
worin (C₁-C₆)-Alkyl seinerseits mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
wobei (C₃-C₇)-Cycloalkyl mit 1 oder 2 Substituenten unabhängig voneinander ausgewählt aus der Gruppe (C₁-C₄)-Alkyl, (C₁-C₄)-Alkoxy, Hydroxy, Amino und Oxo substituiert sein kann,
- R²: für Phenyl steht,
wobei Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein können,
- R³: für Wasserstoff oder (C₁-C₄)-Alkoxy steht,
- R⁴: für (C₁-C₆)-Alkylcarbonyl, Hydroxycarbonyl, (C₁-C₆)-Alkoxycarbonyl, Mono-(C₁-C₆)-Alkylaminocarbonyl oder Di-(C₁-C₆)-Alkylaminocarbonyl steht, wobei (C₁-C₆)-Alkylcarbonyl, (C₁-C₆)-Alkoxycarbonyl, Mono-(C₁-C₆)-Alkylamino-carbonyl und Di-(C₁-C₆)-Alkylaminocarbonyl mit 1 bis 4 Substituenten unabhängig voneinander ausgewählt aus der Gruppe Halogen, Trifluormethyl, (C₁-C₆)-Alkyl und Phenyl substituiert sein können,
worin Phenyl mit 1 oder 2 Substituenten unabhängig voneinander ausgewählt aus der Gruppe Halogen, Cyano, Trifluormethyl, Hydroxy-(C₁-C₄)-Alkyl, (C₁-C₄)-Alkyl, Trifluormethoxy und (C₁-C₄)-Alkoxy substituiert sein kann,
- R⁵: für Halogen, Trifluormethyl, (C₁-C₄)-Alkyl, Trifluormethoxy oder (C₁-C₄)-Alkoxy steht,
- n: für eine Zahl 0, 1 oder 2 steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die vorliegende Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, *N*-Methyl-morpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.
Cycloalkyl steht in Rahmen der Erfindung für einen monocyclischen, gesättigten Alkylrest mit 3 bis 7 bzw. 3 bis 6 Kohlenstoffatomen. Beispielhaft und vorzugsweise seien genannt: Cyclopropyl, Cyclobutyl, Cyclopentyl, Cyclohexyl und Cycloheptyl.
Alkenyl steht im Rahmen der Erfindung für einen linearen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkinyl steht im Rahmen der Erfindung für einen linearen oder verzweigten Alkinylrest mit 2 bis 6 bzw. 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.
Alkylcarbonl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkoxy steht im Rahmen der Erfindung für einen linearen oder verzweigten Alkoxyrest mit 1 bis 6 bzw. 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.
Alkoxycarbonyl stehen im Rahmen der Erfindung für einen linearen oder verzweigten Alkoxyrest mit 1 bis 6 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino, tert.-Butylamino, n-Pentylamino und n-Hexylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl, *tert*.-Butylaminocarbonyl, *n*-Pentylaminocarbonyl und *n-*Hexylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N*-Dimethylaminocarbonyl, *N,N*-Diethylaminocarbonyl, *N-*Ethyl-*N*-methylaminocarbonyl, *N*-Methyl-*N-n*-propylaminocarbonyl, *N-n*-Butyl-*N-*methyl-aminocarbonyl, *N*-tert.-Butyl-*N*-methylaminocarbonyl, *N*-*n*-Pentyl-*N*-methylaminocarbonyl und *N-n*-Hexyl-*N*-methylaminocarbonyl.
Halogen schließt im Rahmen der Erfindung Fluor, Chlor, Brom und Iod ein. Bevorzugt sind Chlor oder Fluor.
Eine Oxo-Gruppe steht im Rahmen der Erfindung für ein Sauerstoffatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind im Rahmen der vorliegenden Erfindung auch Verbindungen der allgemeinen Formel (I-A) 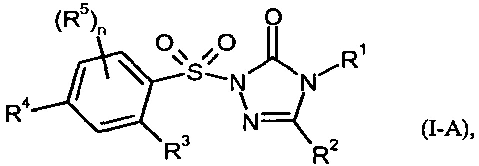 in welcher
- R¹: für Wasserstoff, (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Halogen, Cyano, Oxo, Trifluormethyl, (C₃-C₇)-Cycloalkyl, Phenyl, -OR¹⁰, -NR¹¹R¹², -C(=O)-OR¹³ und -C(=O)-NR¹⁴R¹⁵ substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹², R¹³, R¹⁴ und R¹⁵ unabhängig voneinander jeweils für Wasserstoff oder (C₁-C₆)-Alkyl stehen,
worin (C₁-C₆)-Alkyl seinerseits mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
wobei (C₃-C₇)-Cycloalkyl mit 1 oder 2 Substituenten unabhängig voneinander ausgewählt aus der Gruppe (C₁-C₄)-Alkyl, (C₁-C₄)-Alkoxy, Hydroxy, Amino und Oxo substituiert sein kann,
- R²: für Phenyl steht,
wobei Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein können,
- R³: für Wasserstoff oder (C₁-C₄)-Alkoxy steht,
- R⁴: für (C₁-C₆)-Alkylcarbonyl, (C₁-C₆)-Alkoxycarbonyl, Mono-(C₁-C₆)-Alkylaminocarbonyl oder Di-(C₁-C₆)-Alkylaminocarbonyl steht,
wobei (C₁-C₆)-Alkylcarbonyl, (C₁-C₆)-Alkoxycarbonyl, Mono-(C₁-C₆)-Alkylamino-carbonyl und Di-(C₁-C₆)-Alkylaminocarbonyl mit 1 bis 4 Substituenten unabhängig voneinander ausgewählt aus der Gruppe Halogen, (C₁-C₆)-Alkyl und Phenyl substituiert sein können,
worin Phenyl mit 1 oder 2 Substituenten unabhängig voneinander ausgewählt aus der Gruppe Halogen, Cyano, Trifluormethyl, Hydroxy-(C₁-C₄)-Alkyl, (C₁-C₄)-Alkyl, Trifluormethoxy und (C₁-C₄)-Alkoxy substituiert sein kann,
- R⁵: für Halogen, Trifluormethyl, (C₁-C₄)-Alkyl, Trifluormethoxy oder (C₁-C₄)-Alkoxy steht,
- n: für eine Zahl 0, 1 oder 2 steht,
sowie ihre Salze, Solvate und Solvate der Salze.

Bevorzugt im Rahmen der vorliegenden Erfindung sind Verbindungen der Formel (I), in welcher
- A: für -CH₂- oder -SO₂- steht,
- R¹: für Wasserstoff, (C₁-C₆)-Alkyl, (C₂-C₄)-Alkenyl oder (C₃-C₆)-Cycloalkyl steht, wobei (C₁-C₆)-Alkyl und (C₂-C₄)-Alkenyl mit 1 bis 3 Substituenten unabhängig voneinander ausgewählt aus der Gruppe Fluor, Chlor, Oxo, Trifluormethyl, (C₃-C₆)-Cycloalkyl, Phenyl, -OR¹⁰, -NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, (C₁-C₄)-Alkyl, Trifluormethyl, Hydroxy, (C₁-C₄)-Alkoxy, Trifluormethoxy, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹² , R¹³, R¹⁴ und R¹⁵ unabhängig voneinander jeweils für Wasserstoff oder (C₁-C₆)-Alkyl stehen,
- R²: für Phenyl steht,
wobei Phenyl mit 1 bis 3 Substituenten unabhängig voneinander ausgewählt aus der Gruppe Fluor, Chlor, Cyano, Methyl, Ethyl, Trifluormethyl, Hydroxy, Methoxy, Ethoxy und Trifluormethoxy substituiert sein kann,
- R³: für Wasserstoff oder Methoxy steht,
- R⁴: für (C₁-C₆)-Alkylcarbonyl, Hydroxycarbonyl, (C₁-C₆)-Alkoxycarbonyl, Mono-(C₁-C₆)-Alkylaminocarbonyl oder Di-(C₁-C₆)-Alkylaminocarbonyl steht, wobei Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe Fluor, Trifluormethyl, (C₁-C₄)-Alkyl und Phenyl substituiert sein können,
worin Phenyl mit 1 oder 2 Substituenten unabhängig voneinander ausgewählt aus der Gruppe Fluor, Chlor, Trifluormethyl, Methyl, Ethyl, Trifluormethoxy, Methoxy und Ethoxy substituiert sein kann,
- R⁵: für Fluor, Chlor, Trifluormethyl, Methyl, Ethyl, Trifluormethoxy, Methoxy oder Ethoxy steht,
- n: für eine Zahl 0 oder 1 steht,
sowie ihre Salze, Solvate und Solvate der Salze.

Bevorzugt im Rahmen der vorliegenden Erfindung sind auch Verbindungen der Formel (I-A), in welcher
- R¹: für Wasserstoff, (C₁-C₆)-Alkyl, (C₂-C₄)-Alkenyl oder (C₃-C₆)-Cycloalkyl steht,
wobei (C₁-C₆)-Alkyl und (C₂-C₄)-Alkenyl mit 1 bis 3 Substituenten unabhängig voneinander ausgewählt aus der Gruppe Fluor, Chlor, Oxo, Trifluormethyl, (C₃-C₆)-Cycloalkyl, Phenyl, -OR¹⁰, -NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, (C₁-C₄)-Alkyl, Trifluormethyl, Hydroxy, (C₁-C₄)-Alkoxy, Trifluormethoxy, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹², R¹³, R¹⁴ und R¹⁵ unabhängig voneinander jeweils für Wasserstoff oder (C₁-C₆)-Alkyl stehen,
- R²: für Phenyl steht,
wobei Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein kann,
- R³: für Wasserstoff oder (C₁-C₄)-Alkoxy steht,
- R⁴: für (C₁-C₆)-Alkylcarbonyl, (C₁-C₆)-Alkoxycarbonyl, Mono-(C₁-C₆)-Alkylaminocarbonyl oder Di-(C₁-C₆)-Alkylaminocarbonyl steht,
wobei Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe (C₁-C₆)-Alkyl und Phenyl substituiert sein können,
worin Phenyl mit 1 oder 2 Substituenten unabhängig voneinander ausgewählt aus der Gruppe Fluor, Chlor, Trifluormethyl, (C₁-C₄)-Alkyl, Trifluormethoxy und (C₁-C₄)-Alkoxy substituiert sein kann,
- R⁵: für Fluor, Chlor, Trifluormethyl, Methyl, Ethyl, Trifluormethoxy, Methoxy oder Ethoxy steht,
- n: für eine Zahl 0 oder 1 steht,
sowie ihre Salze, Solvate und Solvate der Salze.

Besonders bevorzugt im Rahmen der vorliegenden Erfindung sind Verbindungen der Formel (I), in welcher
- A: für -CH₂- oder -SO₂- steht,
- R¹: für Wasserstoff, (C₁-C₆)-Alkyl, (C₂-C₄)-Alkenyl oder Cyclopropyl steht,
wobei (C₁-C₆)-Alkyl und (C₂-C₄)-Alkenyl mit 1 bis 3 Substituenten unabhängig voneinander ausgewählt aus der Gruppe Fluor, Oxo, Trifluormethyl, Phenyl, -OR¹⁰,-NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, Methyl, Ethyl, Trifluormethyl, Hydroxy, Methoxy, Ethoxy, Trifluormethoxy, Hydroxycarbonyl, Methoxycarbonyl und Ethoxycarbonyl substituiert sein kann,
und
worin R¹⁰, R¹¹, R¹², R¹³, R¹⁴ und R¹⁵ unabhängig voneinander jeweils für Wasserstoff oder Methyl stehen,
- R²: für
Phenyl steht,
wobei Phenyl mit 1 oder 2 Substituenten unabhängig voneinander ausgewählt aus der Gruppe Fluor, Chlor, Methyl, Ethyl, Trifluormethyl, Hydroxy, Methoxy, Ethoxy und Trifluormethoxy substituiert sein kann,
- R³: für Wasserstoff oder Methoxy steht,
- R⁴: für Methylcarbonyl, Ethylcarbonyl, Methoxycarbonyl, Ethoxycarbonyl, Mono-(C₁-C₆)-Alkylaminocarbonyl oder Di-(C₁-C₆)-Alkylaminocarbonyl steht,
wobei Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe Fluor, Trifluormethyl, Methyl, Ethyl und Phenyl substituiert sein können,
worin Phenyl mit 1 oder 2 Substituenten unabhängig voneinander ausgewählt aus der Gruppe Fluor, Chlor, Trifluormethyl, Methyl, Ethyl, Trifluormethoxy, Methoxy und Ethoxy substituiert sein kann,
- R⁵: für Fluor, Chlor oder Trifluormethyl steht,
- n: für eine Zahl 0 oder 1 steht,
sowie ihre Salze, Solvate und Solvate der Salze.

Besonders bevorzugt im Rahmen der vorliegenden Erfindung sind Verbindungen der Formel (I-A), in welcher
- R¹: für Wasserstoff, (C₁-C₆)-Alkyl, (C₂-C₄)-Alkenyl oder Cyclopropyl steht,
wobei (C₁-C₆)-Alkyl und (C₂-C₄)-Alkenyl mit 1 bis 3 Substituenten unabhängig voneinander ausgewählt aus der Gruppe Fluor, Oxo, Trifluormethyl, Phenyl, -OR¹⁰,-NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, Methyl, Ethyl, Trifluormethyl, Hydroxy, Methoxy, Ethoxy, Trifluormethoxy, Hydroxycarbonyl, Methoxycarbonyl und Ethoxycarbonyl substituiert sein kann,
und
worin R¹⁰, R¹¹, R¹², R¹³, R¹⁴ und R¹⁵ unabhängig voneinander jeweils für Wasserstoff oder (C₁-C₄)-Alkyl stehen,
- R²: für Phenyl steht,
wobei Phenyl mit 1 oder 2 Substituenten unabhängig voneinander ausgewählt aus der Gruppe Fluor, Chlor, Methyl, Ethyl, Trifluormethyl, Hydroxy, Methoxy, Ethoxy und Trifluormethoxy substituiert sein kann,
- R³: für Wasserstoff oder (C₁-C₄)-Alkoxy steht,
- R⁴: für Methylcarbonyl, Ethylcarbonyl, Methoxycarbonyl, Ethoxycarbonyl, Mono-(C₁-C₆)-Alkylaminocarbonyl oder Di-(C₁-C₄)-Alkylaminocarbonyl steht,
wobei Mono-(C₁-C₆)-Alkylaminocarbonyl und Di-(C₁-C₄)-Alkylaminocarbonyl mit 1 bis 4 Substituenten unabhängig voneinander ausgewählt aus der Gruppe Methyl, Ethyl und Phenyl substituiert sein können,
worin Phenyl mit 1 oder 2 Substituenten unabhängig voneinander ausgewählt aus der Gruppe Fluor, Trifluormethyl, (C₁-C₄)-Alkyl, Trifluormethoxy, Methoxy und Ethoxy substituiert sein kann,
sowie ihre Salze, Solvate und Solvate der Salze.

Bevorzugt sind auch Verbindungen der Formel (I), in welcher A für -CH₂- steht.

Bevorzugt sind auch Verbindungen der Formel (I), in welcher A für -SO₂- steht.

Bevorzugt sind auch Verbindungen der Formel (I), in welcher
- R⁴: für Mono-(C₁-C₆)-Alkylaminocarbonyl oder Di-(C₁-C₆)-Alkylaminocarbonyl steht,
wobei Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe Fluor, Trifluormethyl und Phenyl substituiert sein können,
worin Phenyl mit 1 oder 2 Substituenten unabhängig voneinander ausgewählt aus der Gruppe Fluor, Chlor, Trifluormethyl, Methyl, Ethyl, Trifluormethoxy, Methoxy und Ethoxy substituiert ist.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man
[A] eine Verbindung der Formel (II) 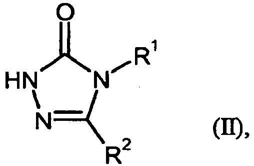 in welcher R¹ und R² jeweils die oben angegebenen Bedeutungen haben,
   in einem inerten Lösungsmittel in Gegenwart einer geeigneten Base mit einer Verbindung der Formel (III) 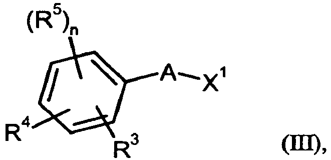 in welcher A, n, R³, R⁴ und R⁵ jeweils die oben angegebenen Bedeutungen haben,
   und
   - X¹: für Halogen, insbesondere für Chlor, steht,
   umsetzt
   oder
[B] von einer Verbindung der Formel (IV) 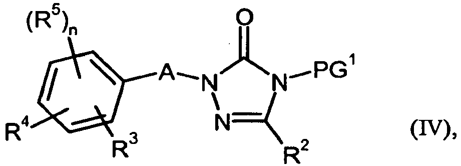 in welcher A, n, R², R³, R⁴ und R⁵ jeweils die oben angegebenen Bedeutungen haben,
   und
   - PG¹: für eine geeignete Schutzgruppe, beispielsweise Allyl, steht,
   in einem inerten Lösungsmittel nach Standardmethoden die Schutzgruppe PG¹ abspaltet
   und die resultierende Verbindung der Formel (V) 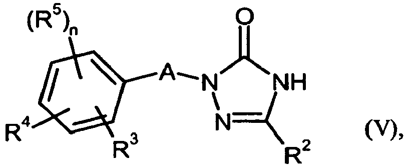 in welcher A, n, R², R³, R⁴ und R⁵ jeweils die oben angegebenen Bedeutungen haben,
   in Gegenwart einer geeigneten Base mit einer Verbindung der Formel (VI)

   R¹-X² (VI),

   in welcher R¹ die oben angegebene Bedeutung hat,
   und
   - X²: für eine Abgangsgruppe, wie beispielsweise Halogen, Mesylat oder Tosylat, steht,
   umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

Die Herstellung der erfindungsgemäßen Verbindungen kann durch die folgenden Syntheseschemata veranschaulicht werden:

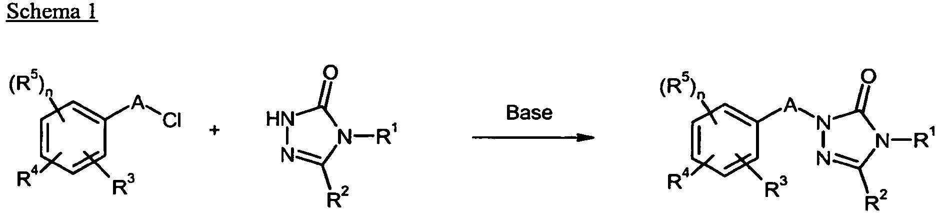

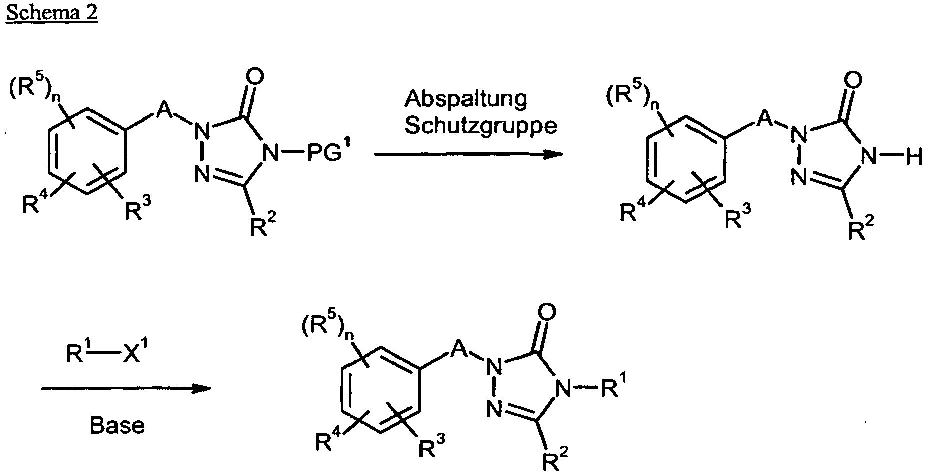

Inerte Lösungsmittel für den Verfahrensschritt (II) + (III) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N,N*-Dimethylformamid (DMF), *N,N*'-Dimethylpropylenharnstoff (DMPU) oder *N*-Methylpyrrolidon (NMP). Ebenso ist es möglich, Gemische der genannten Lösungsmittel zu verwenden. Bevorzugt sind Aceton, Tetrahydrofuran, Dimethylformamid oder Gemische dieser Lösungsmittel.

Als Basen für den Verfahrensschritt (II) + (III) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Cäsiumcarbonat, Alkali-Alkoholate wie Natrium- oder Kaliummethanolat, Natrium- oder Kaliumethanolat oder Lithium-, Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie *N,N*-Diisopropylethylamin, 1,5-Diazabicyclo[4.3.0]non-5-en (DBN) oder 1,8-Diazabicyclo[5.4.0]undec-7-en (DBU). Bevorzugt werden Natriumhydrid und Kalium-tert.-butylat verwendet.

Die Base wird hierbei in einer Menge von 1 bis 5 Mol, bevorzugt in einer Menge von 1 bis 2.5 Mol, bezogen auf 1 Mol der Verbindung der Formel (IV), eingesetzt. Die Reaktion erfolgt im Allgemeinen in einem Temperaturbereich von -78°C bis +50°C, bevorzugt bei -78°C bis +30°C. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Inerte Lösungsmittel für den Verfahrensschritt (V) + (VI) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Acetonitril, Pyridin, Dimethylsulfoxid, *N*,*N*-Dimethylformamid (DMF), *N,N*'-Dimethylpropylenharnstoff (DMPU) oder *N*-Methylpyrrolidon (NMP). Ebenso ist es möglich, Gemische der genannten Lösungsmittel zu verwenden. Bevorzugt wird Dimethylformamid verwendet.

Die Abspaltung der Schutzgruppe im Verfahrensschritt (IV) → (V) erfolgt nach dem dem Fachmann bekannten Methoden [vgl. z. B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Im Fall, dass PG¹ für Allyl steht, erfolgt die Abspaltung vorzugsweise mit Ameisensäure in Dioxan oder Dimethylformamid in Gegenwart eines geeigneten Palladiumkatalysators wie beispielsweise Tetrakis(triphenylphosphin)palladium(0) und einer Amin-Base wie beispielsweise Triethylamin.

Als Basen für den Verfahrensschritt (V) + (VI) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Cäsiumcarbonat, Alkali-Alkoholate wie Natrium- oder Kaliummethanolat, Natrium- oder Kaliumethanolat oder Lithium-, Natrium- oder Kalium-tert.-butylat, Alkalihydride wie Natrium- oder Kaliumhydrid, Amide wie Natriumamid, Lithium- oder Kalium-bis(trimethylsilyl)amid oder Lithiumdiisopropylamid, oder organische Amine wie *N,N-*Diisopropylethylamin, 1,5-Diazabicyclo[4.3.0]non-5-en (DBN) und 1,8-Diazabicyclo[5.4.0]undec-7-en (DBU). Bevorzugt wird Cäsiumcarbonat verwendet.

Die Base wird hierbei in einer Menge von 1 bis 5 Mol, bevorzugt in einer Menge von 1 bis 2.5 Mol, bezogen auf 1 Mol der Verbindung der Formel (IV), eingesetzt. Die Reaktion erfolgt im Allgemeinen in einem Temperaturbereich von 0°C bis +100°C, bevorzugt bei +25°C bis +80°C. Die Umsetzung kann bei normalem, erhöhtem oder bei erniedrigtem Druck durchgeführt werden (z.B. von 0.5 bis 5 bar), gegebenenfalls in einer Mikrowelle. Im Allgemeinen arbeitet man bei Normaldruck.

Weitere erfindungsgemäße Verbindungen können gegebenenfalls auch hergestellt werden durch Umwandlungen von funktionellen Gruppen einzelner Substituenten, insbesondere den unter R⁴ aufgeführten, ausgehend von den nach obigen Verfahren erhaltenen Verbindungen der Formel (I). Diese Umwandlungen werden nach üblichen, dem Fachmann bekannten Methoden bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, insbesondere Bildung von Carbonsäureamiden, sowie Einführung und Entfernung temporärer Schutzgruppen.

Das folgende Syntheseschema soll diese Umwandlungen beispielhaft verdeutlichen:

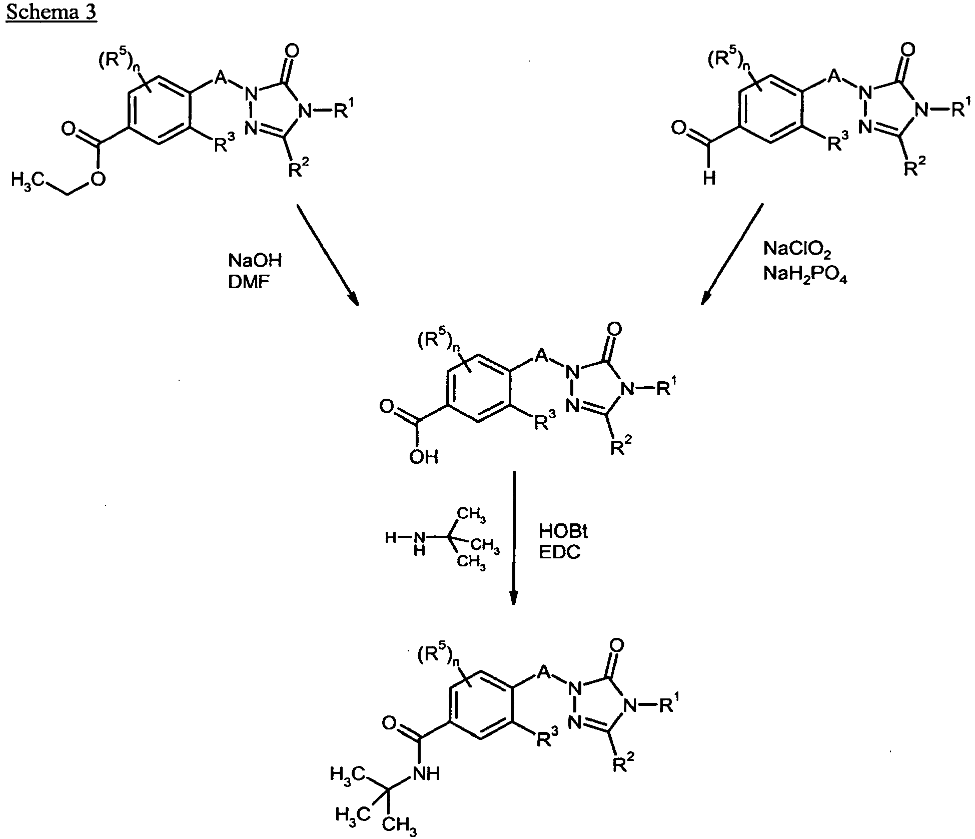

Als Kondensationsmittel für die Amidbildung eignen sich beispielsweise Carbodiimide wie *N,N'-*Diethyl-, *N,N*'-Dipropyl-, *N,N*'-Diisopropyl-, *N,N*'-Dicyclohexylcarbodiimid (DCC) oder N-(3-Dimethylaminoisopropyl)-*N*'-ethylcarbodimid-Hydrochlorid (EDC), Phosgen-Derivate wie *N,N'-*Carbonyldiimidazol (CDI), 1,2-Oxazoliumverbindungen wie 2-Ethyl-5-phenyl-1,2-oxazolium-3-sulfat oder 2-*tert*.-Butyl-5-methyl-isoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidi-no)phosphonium-hexafluorophosphat (PyBOP), *O*-(Benzotriazol-1-yl)-*N,N,N',N*'-tetramethyl-uronium-tetrafluoroborat (TBTU), *O*-(Benzotriazol-1-yl)-*N,N*,*N*',*N*'-tetramethyluronium-hexa-fluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N*',*N'*-tetramethyluronium-hexafluorophosphat (HATU) oder *O-*(1*H-*6-Chlorbenzotriazol-1-yl)-1,1,3,3-tetramethyluronium-tetrafluoroborat (TBTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N-*Diisopropylethylamin. Bevorzugt wird EDC in Kombination mit HOBt oder TBTU in Verbindung mit *N,N-*Diisopropylethylamin verwendet.

Die Amidbildung wird im Allgemeinen in einem Temperaturbereich von -20°C bis +60°C, bevorzugt bei 0°C bis +40°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindungen der Formel (II) sind literaturbekannt oder können in Analogie zur literturbekannten Verfahren hergestellt werden [vgl. z.B. US 3,780,052, EP 422469, EP 425948, EP 507171, EP 703224 und WO 2007/134862].

Die Verbindungen der Formeln (III) und (VI) sind kommerziell erhältlich, literaturbekannt, können in Analogie zu literaturbekannten Verfahren, oder wie im folgenden Syntheseschema (Schema 5) beispielhaft gezeigt, hergestellt werden.

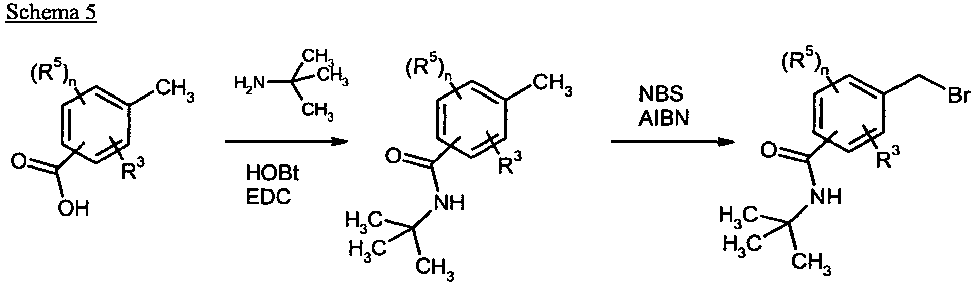

Die Amidkupplung wird unter oben genannten Raktionsbedingungen durchgeführt.

Als Halogenierungsmittel eignen sich elementares Brom mit Essigsäure, 1,3-Dibrom-5,5-dimethylhydantoin sowie insbesondere *N*-Bromsuccinimid (NBS), *N*-Iodsuccinimid (NIS), gegebenenfalls unter Zusatz von α,α'-Azobis(isobutyronitril) (AIBN) als Initiator.

Verbindungen der Formel (IV) können hergestellt werden, indem man ein Hydrazid der Formel (VII) mit einem Isocyanat der Formel (VIII) oder einem Nitrophenylcarbamat der Formel (IX) zu einer Verbindung der Formel (X) umsetzt, diese anschliessend baseninduziert zu einem Triazolon der Formel (XI) cyclisiert, und dieses mit einer Verbindung (III) umsetzt (Schema 4):

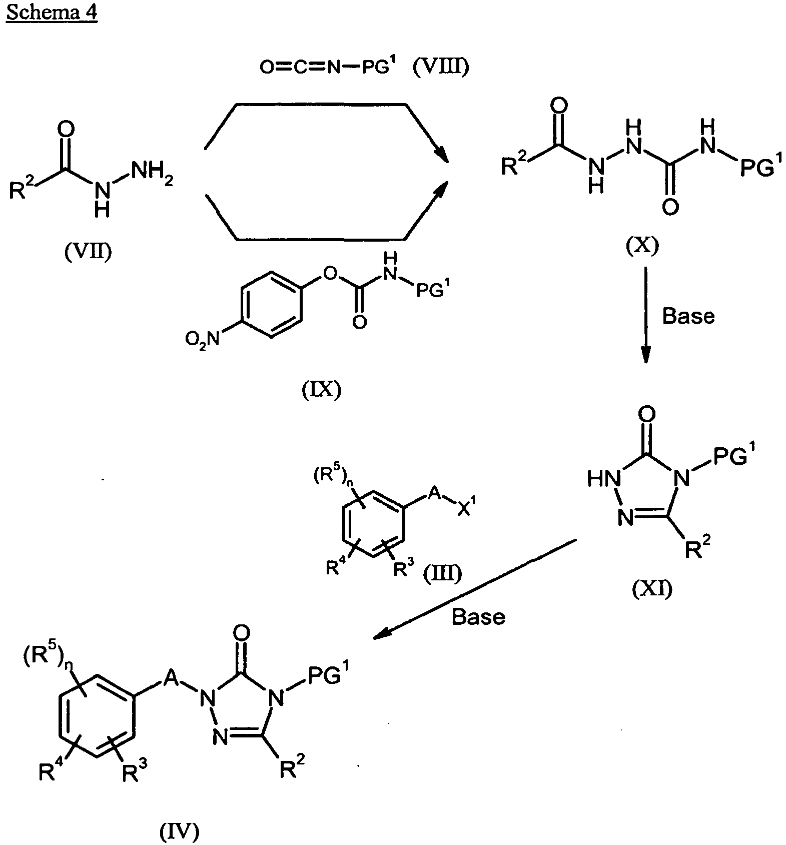

Die erfindungsgemäßen Verbindungen besitzen wertvolle pharmakologische Eigenschaften und können zur Vorbeugung und/oder Behandlung von verschiedenen Erkrankungen und krankheitsbedingten Zuständen bei Menschen und Tieren verwendet werden.

Bei den erfindungsgemäßen Verbindungen handelt es sich um potente selektive V2- und/ oder um duale V1a/V2-Rezeptor-Antagonisten, die die Vasopressin-Aktivität *in vitro* und *in vivo* inhibieren. Darüber hinaus wirken die erfindungsgemäßen Verbindungen auch als Antagonisten am verwandten Oxytozin-Rezeptor.

Die erfindungsgemäßen Verbindungen sind insbesondere für die Prophylaxe und/oder Behandlung von kardiovaskulären Erkrankungen geeignet. In diesem Zusammenhang seien als Zielindikationen beispielhaft und vorzugsweise genannt: akute und chronische Herzinsuffizienz, arterielle Hypertonie, koronare Herzerkrankung, stabile und instabile Angina pectoris, myokardiale Ischämie, Myokardinfarkt, Schock, Arteriosklerose, atriale und ventrikuläre Arrhythmien, transitorische und ischämische Attacken, Hirnschlag, entzündliche kardiovaskuläre Erkrankungen, periphere und kardiale Gefäßerkrankungen, periphere Durchblutungsstörungen, arterielle pulmonale Hypertonie, Spasmen der Koronararterien und peripherer Arterien, Thrombosen, thromboembolische Erkrankungen, Ödembildung wie zum Beispiel pulmonales Ödem, Himödem, renales Ödem oder Herzinsuffizienz-bedingtes Ödem, sowie Restenosen wie nach Thrombolysetherapien, percutan-transluminalen Angioplastien (PTA), transluminalen Koronarangioplastien (PTCA), Herztransplantationen und Bypass-Operationen.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Weiterhin eignen sich die erfindungsgemäßen Verbindungen zur Verwendung als Diuretikum für die Behandlung von Ödemen und bei Elektrolytstörungen, insbesondere bei der hypervolämischen und euvolämischen Hyponaträmie.

Die erfindungsgemäßen Verbindungen sind weiter geeignet für die Prophylaxe und/oder Behandlung der polyzystischen Nierenkrankheit (PCKD) und des Syndroms der inadäquaten ADH-Sekretion (SIADH).

Außerdem können die erfindungsgemäßen Verbindungen für die Prophylaxe und/oder Behandlung der Leberzirrhose, des Aszites, von Diabetes mellitus und diabetischen Folgeerkrankungen, wie z.B. Neuropathie und Nephropathie, von akutem und chronischem Nierenversagen sowie von chronischer Niereninsuffizienz verwendet werden.

Ferner eignen sich die erfindungsgemäßen Verbindungen für die Prophylaxe und/oder Behandlung zentralnervöser Störungen wie Angstzuständen und Depressionen, von Glaukom sowie von Krebs, insbesondere von Lungentumoren.

Darüber hinaus können die erfindungsgemäßen Verbindungen für die Prophylaxe und/oder Behandlung von entzündlichen Erkrankungen, asthmatischen Erkrankungen, chronisch-obstruktiven Atemwegserkrankungen (COPD), Schmerzzuständen, Prostatahypertrophien, Inkontinenz, Blasenentzündung, hyperaktiver Blase, Erkrankungen der Nebenniere wie zum Beispiel Phäochromozytom und Nebennierenapoplexie, Erkrankungen des Darms wie zum Beispiel Morbus Crohn und Diarrhoe, oder von Menstruationsstörungen wie zum Beispiel Dysmenorrhöen eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, die mindestens eine der erfindungsgemäßen Verbindungen sowie einen oder mehrere weitere Wirkstoffe enthalten, insbesondere zur Behandlung und/oder Prophylaxe der zuvor genannten Erkrankungen. Als hierfür geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Diuretika, insbesondere Schleifendiuretika sowie Thiazide und Thiazid-ähnliche Diuretika;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Calcium-Sensitizer, wie beispielhaft und vorzugsweise Levosimendan;
- NO- und Häm-unabhängige Aktivatoren der Guanylatcyclase, wie insbesondere Cinaciguat sowie die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der Guanylatcyclase, wie insbesondere Riociguat sowie die in WO 00/06568, WO 00/06569, WO 02/42301 und WO 03/095451 beschriebenen Verbindungen;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib;
- den Energiestoffwechsel des Herzens beeinflussende Verbindungen, wie beispielhaft und vorzugsweise Etomoxir, Dichloracetat, Ranolazine oder Trimetazidine;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten und Rho-Kinase-Inhibitoren; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Vasopeptidase-Inhibitoren, Inhibitoren der neutralen Endopeptidase, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embusartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Vasopeptidase-Inhibitor bzw. Inhibitor der neutralen Endopeptidase (NEP), wie beispielhaft und vorzugsweise Omapatrilat oder AVE-7688, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon, Canrenon oder Kalium-Canrenoat, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 10 mg/kg, vorzugsweise etwa 0.01 bis 1 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- Alk: Alkyl
- Boc: *tert*.-Butoxycarbonyl
- Br.s: breites Singulett (bei NMR)
- CI: chemische Ionisation (bei MS)
- DCI: direkte chemische Ionisation (bei MS)
- DME: 1,2-Dimethoxyethan
- DMF: Dimethylformamid
- DMSO: Dimethylsulfoxid
- d. Th.: der Theorie (bei Ausbeute)
- EDC: *N*'-(3-Dimethylaminopropyl)-*N*-ethylcarbodiimid (-Hydrochlorid)
- EE: Essigsäureethylester
- eq.: Äquivalent(e)
- ESI: Elektrospray-Ionisation (bei MS)
- FMOC: 9-Fluorenylmethoxycarbonyl
- GC/MS: Gaschromatographie-gekoppelte Massenspektrometrie
- ges.: gesättigt
- h: Stunde(n)
- Hal: Halogen
- HOBt: 1-Hydroxy-1*N*-benzotriazol-Hydrat
- HPLC: Hochdruck-, Hochleistungsflüssigchromatographie
- konz.: konzentriert
- LC/MS: Flüssigchromatographie-gekoppelte Massenspektrometrie
- LDA: Lithiumdiisopropylamid
- LiHMDS: Lithiumhexamethyldisilazan
- min: Minute(n)
- MS: Massenspektrometrie
- NMR: Kernresonanzspektrometrie
- PG: Schutzgruppe
- rac: racemisch / Racemat
- R_{f}: Retentionsfaktor (bei Dünnschichtchromatographie auf Kieselgel)
- RT: Raumtemperatur
- Rₜ: Retentionszeit (bei HPLC)
- TBTU: *O*-(Benzotriazol-1-yl)-*N,N,N',N*'-tetramethyluronium-Tetrafluoroborat
- THF: Tetrahydrofuran
- TMOF: Trimethylorthoformiat
- UV: Ultraviolett-Spektrometrie
- v/v: Volumen zu Volumen-Verhältnis (einer Lösung)

### LC/MS-, HPLC- und GC/MS-Methoden:

### Methode 1 (HPLC):

Instrument: HP 1100 mit DAD-Detektion; Säule: Kromasil RP-18, 150 mm x 4.6 mm, 5 µm; Eluent A: 0.5 ml Phosphorsäure (85%ig)/l Wasser, Eluent B: Acetonitril; Gradient: 0 min 10% B, 1.0 min 10% B, 4.0 min 90% B, 5.0 min 90% B, 5.5 min 10% B, 6.0 min 10% B; Fluss: 2.0 ml/min; Ofen: 30°C; UV-Detektion: 210 nm.

### Methode 2 (LC/MS):

Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 3 (LC/MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.

### Methode 4 (LC/MS):

Instrument: Micromass QuattroPremier mit Waters UPLC Acquity; Säule: Thermo Hypersil GOLD 1.9µ 50 x 1mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.

### Methode 5 (präparative HPLC):

Instrument: Abimed Gilson Pump 305/306, Manometric Module 806; Säule: Grom-Sil 120 ODS-4HE 10 µm, 250 mm x 30 mm; Eluent: A = Wasser, B = Acetonitril; Gradient: 0.0 min 10% B, 3 min 10% B, 30 min 95% B, 42 min 95% B, 42.01 min 10% B, 45 min 10% B; Fluss: 50 ml/min; Säulentemperatur: RT; UV-Detektion: 210 nm.

### Methode 6 (LC/MS):

Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 7 (LC/MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (präparative HPLC):

Säule: Grom-Sil 120 ODS-4HE, 10 µm, 250 mm x 30 mm; Eluent A: Wasser, Eluent B: Acetonitril; Gradient: 0.0 min 10% B → 3 min 10% B → 30 min 95% B → 42 min 95% B → 42.01 min 10% B → 45 min 10% B; Fluss: 50 ml/min; Säulentemperatur: RT; UV-Detektion: 210 nm.

### Ausgangsverbindungen und Intermediate:

### Beispiel 1A

### 2-[(4-Chlorphenyl)carbonyl]-N-(prop-2-en-1-yl)hydrazincarboxamid

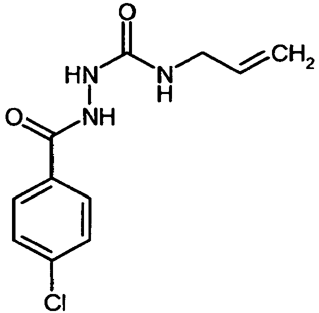

5.00 g (29.3 mmol) 4-Chlorbenzolcarbohydrazid wurden in trockenem Tetrahydrofuran (150 ml) bei 50°C suspendiert, anschließend tropfte man 2.63 ml (29.9 mmol) Allylisocyanat gelöst in 110 ml trockenem Tetrahydrofuran hinzu. Vorübergehend ging das Ausgangsmaterial vollständig in Lösung, dann fiel ein feiner Niederschlag aus. Es wurde 2 h bei 50°C gerührt. Nach Abkühlen auf Raumtemperatur wurde mit Diethylether versetzt. Der farblose Feststoff wurde abgesaugt, mit Diethylether gewaschen und im Hochvakuum getrocknet. 7.42 g (100% d.Th.) der Zielverbindung wurden so erhalten.

HPLC [Methode 1] Rₜ = 3.45 min

LC-MS [Methode 3] Rₜ = 1.51 min; MS [ESIpos]: m/z = 254 (M+H)⁺

¹H-NMR (400 MHz, DMSO-d₆): δ = 3.60 - 3.70 (m, 2H), 5.01 (dd, 1H), 5.14 (dd, 1H), 5.72 - 5.86 (m, 1H), 6.70 (s, 1H), 7.56 (d, 2H), 7.85 - 7.95 (m, 3H), 10.21 (s, 1H).

In analoger Weise wurde die folgende Verbindung erhalten:

| **Beispiel Nr.** | **Struktur** | **LC/MS Rₜ [Methode]** | **HPLC Rₜ [Methode]** |
|---|---|---|---|
| 2A | 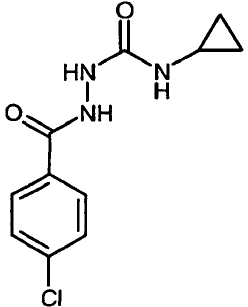 | Rₜ = 0.71 min [4]; MS [ESIpos]: m/z = 254 (M+H)⁺ | Rₜ = 3.332 min |
| | | | [1] |

### Beispiel 3A

### 5-(4-Chlorphenyl)-4-(prop-2-en-1-yl)-2,4-dihydro-3H-1,2,4-triazol-3-on

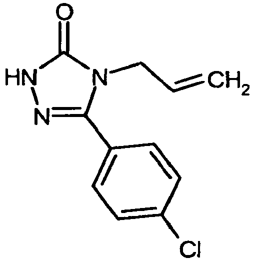

26.8 g (106 mmol) 2-[(4-Chlorphenyl)carbonyl]-N-(prop-2-en-1-yl)hydrazincarboxamid aus Beispiel 1A wurden in 210 ml 3M Natronlauge suspendiert und 20 h unter Rückfluß erhitzt. Nach dem Abkühlen stellte man mit halbkonzentrierter Salzsäure auf pH 10 ein. Der ausgefallene farblose Feststoff wurde abgesaugt, mit Wasser neutral gewaschen und anschließend in Methanol verrührt. Die Mischung wurde durch Filtration von unlöslichen Bestandteilen befreit, das Filtrat am Rotationsverdampfer unter vermindertem Druck eingedampft und der Rückstand im Hochvakuum getrocknet. Man erhielt so 21.5 g (86.4% d. Th.) der gewünschten Verbindung als Feststoff.

HPLC [Methode 1] Rt = 3.80 min;

LC-MS [Methode 3] Rt = 1.79 min; MS [ESIpos]: m/z = 254 (M+H)+ 1H-NMR (400 MHz, DMSO-d6): δ = 4.30 - 4.35 (m, 2H), 4.91 (dd, 1H), 5.11 (dd, 1H), 5.76-5.90 (m, 1H), 7.58 (d, 2H), 7.65 (d, 2H), 12.05 (s, 1H).

In analoger Weise wurde die folgende Verbindung erhalten:

| **Beispiel Nr.** | **Struktur** | **LC/MS Rₜ [Methode]** | **HPLC Rₜ [Methode]** |
|---|---|---|---|
| **4A** | 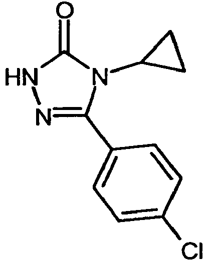 | Rₜ = 1.85 min [3]; MS [ESIpos]: m/z = 236 (M+H)⁺ | Rₜ = 3.725 min |
| | | | [1] |

Die Synthese der folgenden Edukte ist in den genannten Patenten beschrieben.

| **Beispiel Nr.** | **Struktur** | **Patentnummer Patentbeispiel** |
|---|---|---|
| | | **[Jahr]** |
| **5A** | 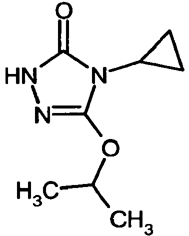 | EP 703224 |
| | | 10 |
| | | [1996] |
| **6A** | 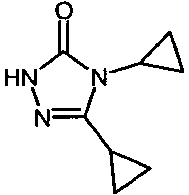 | EP 422469 A2 |
| | | II-36 |
| | | [1991] |
| **7A** | 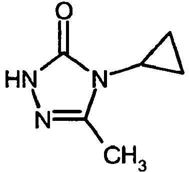 | EP 422469 A2 |
| | | II-18 |
| | | [1991] |
| **8A** | 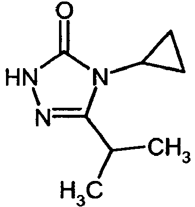 | EP 422469 A2 |
| | | II-27 |
| | | [1991] |
| **9A** | 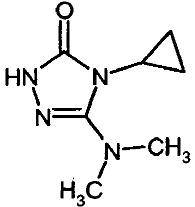 | EP 422469 A2 |
| | | II-91 |
| | | [1991] |
| **10A** | 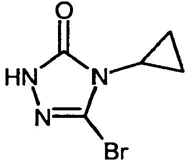 | EP 425948 |
| | | II-3 |
| | | [1991] |
| **11A** | 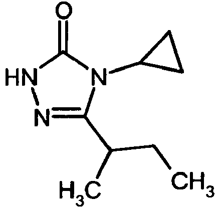 | EP 422469 A2 |
| | | II-32 |
| | | [1991] |
| **12A** | 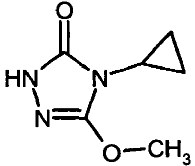 | EP 507171 |
| | | II-5 |
| | | [1992] |
| **13A** | 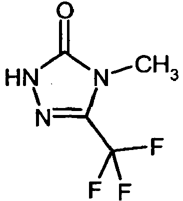 | US 3780052 |
| | | 2 |
| | | [1973] |

### Beispiel 14A

### 4-{[3-(4-Chlorphenyl)-4-cyclopropyl-5-oxo-4,5-dihydro-1H-1,2,4-triazol-1-yl]sulfonyl}benzolcarbaldehyd

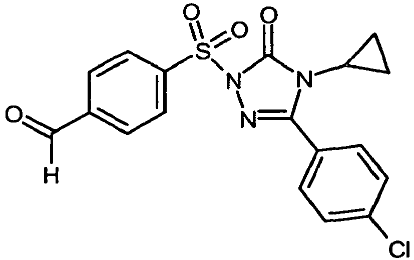

300 mg (1.27 mmol) der Verbindung aus Beispiel 4A wurden in 3 ml DMF gelöst und bei 0°C mit 56 mg (1.40 mmol) Natriumhydrid (60%ig in Paraffinöl) versetzt. Es wurde 30 min bei dieser Temperatur nachgerührt und anschließend mit 260 mg (1.27 mmol) 4-Formylbenzolsulfonylchlorid versetzt. Das Gemisch wurde auf Raumtemperatur erwärmt und man ließ 12 h bei Raumtemperatur nachrühren. Das Reaktionsgemisch wurde mit 15 ml Wasser versetzt, der ausgefallene Feststoff abgesaugt, mit etwas Wasser gewaschen und im Hochvakuum getrocknet. Man erhielt so 351 mg (58% d. Th.) der Zielverbindung.

LC/MS [Methode 3]: Rₜ = 2.36 min; MS [ESIpos]: m/z = 404 (M+H)⁺.

### Beispiel 15A

### 4-{[3-(4-Chlorphenyl)-4-cyclopropyl-5-oxo-4,5-dihydro-1H-1,2,4-triazol-1-yl]sulfonyl}benzolcarbonsäure

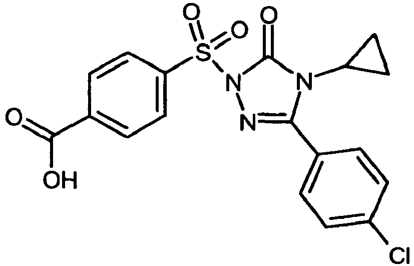

345 mg (0.73 mmol) der Verbindung aus Beispiel 14A sowie 0.58 ml (5.45 mmol) 2-Methyl-2-buten wurden in 3 ml Aceton gelöst und bei RT mit einer Lösung von 376 mg (2.73 mmol) Natriumdihydrogenphosphat-1-hydrat und 460 mg (4.07 mmol) Natriumchlorit in 9 ml Wasser versetzt. Es wurde 16 h bei dieser Temperatur nachgerührt und anschließend mit 15 ml Wasser sowie 15 ml Ethylacetat verdünnt. Nach Trennen der Phasen wurde die wässrige Phase nochmals mit 15 ml Ethylacetat extrahiert, die vereinigten organischen Phasen mit gesättigter Natriumchloridlösung gewaschen und über Magnesiumsulfat getrocknet. Nach Filtration wurde das Solvens eingeengt und der Rückstand im Hochvakuum getrocknet. Man erhielt so 337 mg (104% d. Th.) der Zielverbindung, die ohne weitere Reinigung weiter umgesetzt wurde

LC/MS [Methode 3]: Rt = 2.28 min; MS [ESIpos]: m/z = 419.9 (M+H)+

1H-NMR (400 MHz, CDC13): δ = 0.68- 0.76 (m, 2H), 0.95- 1.02 (m, 2H), 2.88 (m, 1H), 7.48 (d, 2H), 7.70 (d, 2H), 8.23 (s, 4H).

### Beispiel 16A

### Ethyl-N-({2-[(4-chlorphenyl)carbonyl]hydrazinyl}carbonyl)glycinat

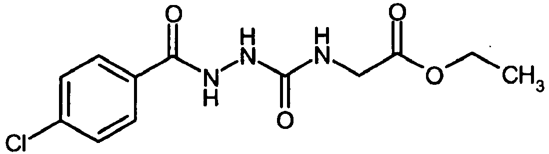

Eine Suspension von 12.95 g (75.9 mmol) 4-Chlorbenzohydrazid in 50 ml trockenem THF wurde bei 50°C vorgelegt und tropfenweise mit einer Lösung von 10.0 g (77.5 mmol) Ethyl-2-isocyanato-acetat in 100 ml trockenem THF versetzt. Zunächst bildete sich eine Lösung, dann fiel ein Niederschlag aus. Nach Ende der Zugabe wurde die Mischung noch 2 h bei 50°C weiter gerührt und dann über Nacht bei RT stehen gelassen. Die Kristalle wurden durch Filtration isoliert, mit wenig Diethylether gewaschen und im Hochvakuum getrocknet. Es wurden 21.43 g (89% d. Th) der Titelverbindung erhalten.

LC/MS [Methode 2]: Rₜ = 1.13 min; m/z = 300 (M+H)⁺

¹H-NMR (DMSO-d₆, 400 MHz): δ = 1.19 (t, 3H), 3.77 (d, 2H), 4.09 (q, 2H), 6.88 (br. s, 1H), 7.57 (d, 2H), 7.91 (d, 2H), 8.21 (s, 1H), 10.29 (s, 1H).

### Beispiel 17A

### [3-(4-Chlorphenyl)-5-oxo-1,5-dihydro-4H-1,2,4-triazol-4-yl]essigsäure

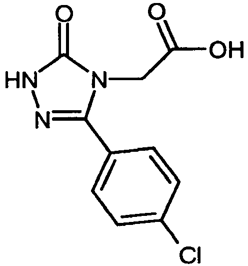

21.43 g (67.9 mmol) der Verbindung aus Beispiel 16A wurden mit 91 ml 3 N Natronlauge versetzt und über Nacht unter Rückfluss erhitzt. Nach Abkühlen auf RT wurde die Mischung durch langsame Zugabe von ca. 20%-iger Salzsäure auf pH 1 eingestellt. Der ausgefallene Feststoff wurde durch Filtration isoliert, mit Wasser gewaschen und bei 60°C im Vakuum getrocknet. Es wurden 17.55 g der Titelverbindung in ca. 88% Reinheit (90% d. Th.) erhalten.

LC/MS [Methode 2]: Rₜ = 0.94 min; m/z = 254 (M+H)⁺

¹H-NMR (DMSO-d₆, 400 MHz): δ = 4.45 (s, 2H), 7.65-7.56 (m, 4H), 12.09 (s, 1H), 13.25 (br. s, 1H).

### Beispiel 18A

### 5-(4-Chlorphenyl)-4-(3,3,3-trifluor-2-oxopropyl)-2,4-dihydro-3H-1,2,4-triazol-3-on (Keton-Form) bzw. 5-(4-Chlorphenyl)-4-(3,3,3-trifluor-2,2-dihydroxypropyl)-2,4-dihydro-3H-1,2,4-triazol-3-on (Hydrat-Form)

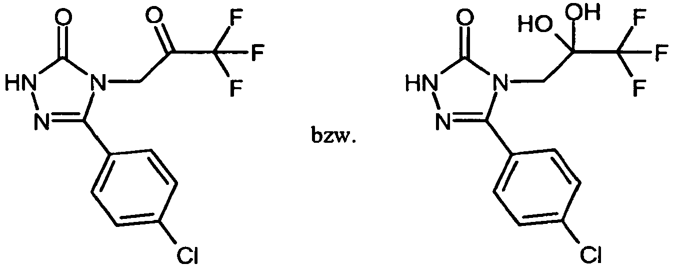

5.0 g (16.36 mmol) der Verbindung aus Beispiel 17A wurden unter Argon in 200 ml Pyridin gelöst und mit 17.18 g (81.8 mmol) Trifluoressigsäureanhydrid versetzt. Dabei stieg die Temperatur auf ca. 35°C an. Nach 30 min wurde das Pyridin am Rotationsverdampfer entfernt und der Rückstand mit 1.5 Liter 0.5 N Salzsäure versetzt. Diese Mischung wurde auf 70°C erwärmt und dann in der Wärme filtriert. Der Feststoff wurde mit etwas Wasser gewaschen. Das gesamte Filtrat wurde dreimal mit Ethylacetat extrahiert. Die vereinigten organischen Phasen wurden nacheinander mit Wasser, gesättigter wässriger Natriumhydrogencarbonat-Lösung und gesättigter wässriger Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.56 g (68% d. Th.) der Titelverbindung in der Hydrat-Form erhalten.

LC/MS [Methode 2]: Rₜ = 1.51 min; m/z = 306 (M+H)⁺ und 324 (M+H)⁺ (Keton- bzw. Hydrat-Form)

¹H-NMR (DMSO-d₆, 400 MHz): δ = 3.98 (s, 2H), 7.61 (d, 2H), 7.68 (br. s, 2H), 7.72 (d, 2H), 12.44 (s, 1H).

### Beispiel 19A

### 5-(4-Chlorphenyl)-4-(3,3,3-trifluor-2-hydroxypropyl)-2,4-dihydro-3H-1,2,4-triazol-3-on

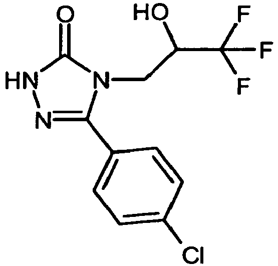

3.56 g (11.0 mmol) der Verbindung aus Beispiel 18A wurden in 100 ml Methanol gelöst und unter Eiskühlung mit 3.75 g (99.5 mmol) Natriumborhydrid versetzt. Nach 1.5 h wurden langsam 200 ml 1 M Salzsäure zugegeben. Das Methanol wurde am Rotationsverdampfer entfernt, der Rückstand mit 500 ml Wasser verdünnt und dreimal mit Ethylacetat extrahiert. Die vereinigten organischen Phasen wurden mit gesättigter wässriger Natriumhydrogencarbonat-Lösung und dann mit gesättigter wässriger Natriumchlorid-Lösung gewaschen, über Natriumsulfat getrocknet und am Rotationsverdampfer vom Lösungsmittel befreit. Der Rückstand wurde im Hochvakuum getrocknet. Es wurden 3.04 g (90% d. Th.) der Titelverbindung erhalten.

LC/MS [Methode 6]: R, = 1.80 min; m/z = 308 (M+H)⁺

¹H-NMR (DMSO-d₆, 400 MHz): δ = 3.77 (dd, 1H), 3.92 (dd, 1H), 4.34-4.23 (m, 1H), 6.85 (d, 1H), 7.62 (d, 2H), 7.75 (d, 2H), 12.11 (s, 1H).

### Beispiel 20A

### Methyl-4-{[3-(4-chlorphenyl)-4-cyclopropyl-5-oxo-4,5-dihydro-1H-1,2,4-triazol-1-yl]methyl}-3-methoxybenzolcarboxylat

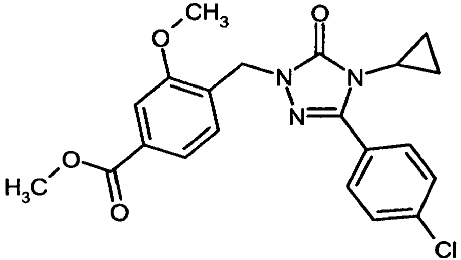

800 mg (3.39 mmol) 5-(4-Chlorphenyl)-4-cyclopropyl-2,4-dihydro-3H-1,2,4-triazol-3-on (Herstellung gemäß WO 2007/134862 Beispiel 36A) und 1.66 g (5.09 mmol) Cäsiumcarbonat wurden in 10 ml Aceton suspendiert und mit 1.14 g (4.41 mmol) Methyl-4-(brommethyl)-3-methoxybenzolcarboxylat versetzt. Es wurde 2 h bei 60°C gerührt. Man verdünnte die Suspension mit Wasser. Es wurde zweimal mit je 15 ml Ethylacetat extrahiert. Die vereinigten organischen Phasen wurden über Natriumsulfat getrocknet und am Rotationsverdampfer vom Lösungsmittel befreit. Nach Reinigung durch Flash-Chromatographie über Kieselgel durch Elution mit Cyclohexan/ Ethylacetat (Gradient 4:1 → 1:1) erhielt man 910 mg (64 % d. Th.) der Zielverbindung.

LC-MS [Methode 4] Rₜ = 1.28 min; MS [ESIpos]: m/z = 414 (M+H)⁺

¹H-NMR (400 MHz, CDCl₃): δ = 0.75- 0.83 (m, 2H), 1.00- 1.07 (m, 2H), 2.97- 3.05 (m, 1H), 3.90 (s, 3H), 3.92 (s, 3H), 5.08 (s, 2H), 7.17 (d, 1H), 7.43 (d, 2H), 7.53 (s, 1H), 7.59 (d, 1H), 7.68 (d, 2H).

### Beispiel 21A

### 4- {[3-(4-Chlorphenyl)-4-cyclopropyl-5-oxo-4,5-dihydro-1H-1,2,4-triazol-1-yl]methyl}-3-methoxybenzolcarbonsäure

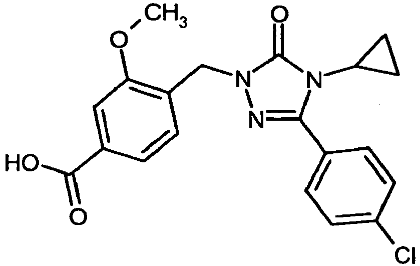

830 mg (2.01 mmol) der Verbindung aus aus Beispiel 20A wurden in 10 ml Tetrahydrofuran/ Methanol (1:1) gelöst und mit 2 ml 2N Natronlauge (4.01 mmol) versetzt. Es wurde eine Stunde bei 80°C gerührt. Man verdünnte die Reaktionslösung mit 10 ml Wasser und extrahierte zweimal mit je 15 ml Ethylacetat. Die vereinigten organischen Phasen enthielten kein Produkt und wurden verworfen. Die wässrige Produktphase wurde mit 1 N Salzsäure angesäuert und der entstehende Niederschlag abfiltriert. Der Feststoff wurde im Hochvakuum getrocknet. Da die Mutterlauge noch viel Produkt enthielt, wurde sie am Rotationsverdampfer vollständig eingeengt. So erhielt man zusammen 747 mg (93 % d. Th.) der Zielverbindung.

LC-MS [Methode 4] Rₜ = 1.11 min; ; MS [ESIpos]: m/z = 400 (M+H)⁺

¹H-NMR (400 MHz, CDCl₃): δ = 0.77- 0.85 (m, 2H), 1.01- 1.08 (m, 2H), 2.99- 3.07 (m, 1H), 3.92 (s, 3H), 5.11 (s, 2H), 7.17 (d, 1H), 7.44 (d, 2H), 7.54 (s, 1H), 7.61 (d, 1H), 7.70 (d, 2H).

### Beispiel 22A

### N-tert.-Butyl-2-methoxy-4-methylbenzamid

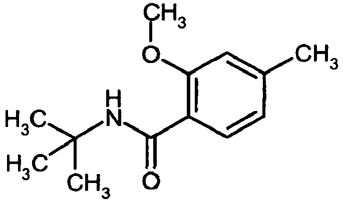

200 mg (1.24 mmol) 2-Methoxy-4-benzoesäure wurden in 3 ml DMF vorgelegt und mit 240 mg (1.57 mmol) HOBt und mit 277 mg (1.44 mmol) EDC versetzt. Nach 10 min Rühren bei RT wurden 139 µl (1.32 mmol) 2-Methylpropan-2-amin hinzugegeben und die Mischung 30 min bei Raumtemperatur weitergerührt. Anschließend wurde die Reaktionslösung mit ca. 5 ml Wasser versetzt und zweimal mit je 10 ml Ethylacetat extrahiert. Die vereinigten organischen Phasen wurden über Natriumsulfat getrocknet, filtriert und im Vakuum eingeengt. Das Rohprodukt wurde chromatograpisch gereinigt [Methode 8]. Es wurden 164 mg (62% d. Th.) der Zielverbindung erhalten.

LC-MS [Methode 2] Rₜ = 1.88 min; MS [ESIpos]: m/z = 222 (M+H)⁺

### Beispiel 23A

### 4-(Brommethyl)-N-tert-butyl-2-methoxybenzamid

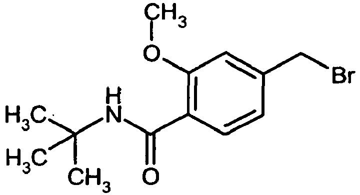

160 mg (0.72 mmol) der Verbindung aus Beispiel 22A wurden mit 129 mg (0.72 mmol) N-Bromsuccinimid sowie 12 mg (0.07 mmol) 2,2'-Azobis-2-methylpropannitril in 5 ml Tetrachlorkohlenstoff 16 h unter Rückfluß erhitzt. Nach Abkühlen auf RT wurde mit 10 ml Dichlormethan verdünnt und mit 10 ml Wasser gewaschen. Es wurde über Natriumsulfat getrocknet, filtriert und im Vakuum eingeengt. Das Rohprodukt wurde anschließend chromatographisch gereinigt [Methode 8]. Es wurden 103 mg (47% d.Th.) der Zielverbindung erhalten.

LC-MS [Methode 7] Rₜ = 2.32 min; MS [ESIpos]: m/z = 300 und 302 (M+H)⁺

¹H-NMR (400MHz, CDCl₃): δ = 1.45 (s, 9H), 3.97 (s, 3H), 4.47 (s, 2H), 6.98 (d, 1H), 7.08 (dd, 1H), 7.75 (br. s., 1H), 8.14 (d, 1H).

### Beispiel 24A

### Methyl-4-{[3-(4-chlorphenyl)-5-oxo-4-(3,3,3-trifluor-2-hydroxypropyl)-4,5-dihydro-1H-1,2,4-triazol-1-yl]methyl}benzoat

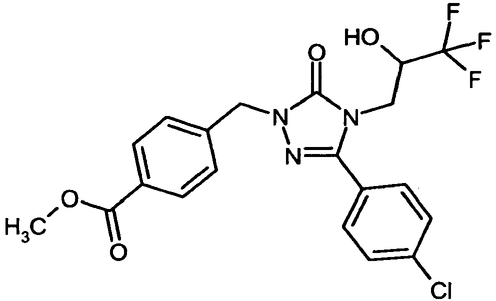

305 mg (0.99 mmol) der Verbindung aus Beispiel 19A und 355 mg (1.09 mmol) Cäsiumcarbonat wurden in 10 ml Aceton suspendiert und mit 227 mg (0.99 mmol) Methyl-4-(brommethyl)benzoat versetzt. Es wurde 3 h bei 50°C gerührt. Man verdünnte die Suspension mit Wasser. Es wurde zweimal mit je 15 ml Ethylacetat extrahiert. Die vereinigten organischen Phasen wurden über Natriumsulfat getrocknet und am Rotationsverdampfer vom Lösungsmittel befreit. Nach Reinigung durch Flash-Chromatographie über Kieselgel durch Elution mit Cyclohexan/ Ethylacetat (Gradient 10:1 → 2:1) erhielt man 284 mg (63 % d. Th.) der Zielverbindung.

LC-MS [Methode 4] Rₜ = 1.28 min; MS [ESIpos]: m/z = 456 (M+H)⁺

¹H-NMR (400MHz, CDCl₃): δ = 3.91 (s, 3H), 3.98 - 4.13 (m, 2H), 4.41 - 4.51 (m, 1H), 4.91 (d, 1H), 5.07 (s, 2H), 7.45 (d, 2H), 7.46 - 7.53 (m, 4H), 8.03 (d, 2H).

### Beispiel 25A

### Methyl-3-{[3-(4-chlorphenyl)-5-oxo-4-(3,3,3-trifluor-2-hydroxypropyl)-4,5-dihydro-1H-1,2,4-triazol-1-yl]methyl}benzoat

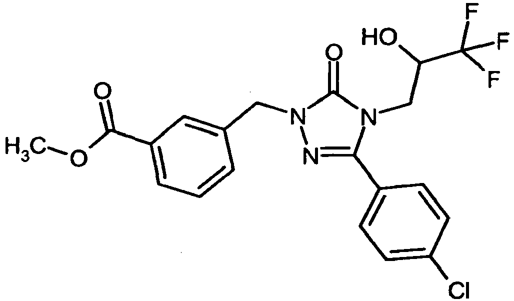

Analog zur Verbindung aus Beispiel 24A wurden 267 mg (0.87 mmol) der Verbindung aus Beispiel 19A mit 199 mg (0.87 mmol) Methyl-3-(brommethyl)benzoat umgesetzt. Es wurden 302 mg (76% d.Th) der Zielverbindung erhalten.

LC-MS [Methode 7] Rₜ = 2.41 min; MS [ESIpos]: m/z = 456 (M+H)⁺

¹H-NMR (400MHz, CDCl₃): δ = 3.91 (s, 3H), 4.01 (dd, 1H), 4.09 (dd, 1H), 4.45 - 4.57 (m, 1H), 5.02 - 5.17 (m, 3H), 7.41 - 7.55 (m, 4H), 7.58 (d, 1H), 7.93 (s, 1H), 7.98 (d, 1H).

### Beispiel 26A

### tert.-Butyl-3-chlor-4-{[3-(4-chlorphenyl)-4-cyclopropyl-5-oxo-4,5-dihydro-1H-1,2,4-triazol-1-yl]methyl}benzoat

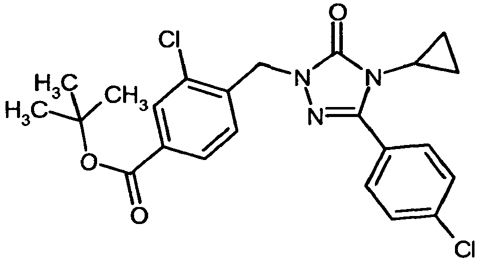

1.00 g (4.22 mmol) 5-(4-Chlorphenyl)-4-cyclopropyl-2,4-dihydro-3H-1,2,4-triazol-3-on (Herstellung gemäß WO 2007/134862 Beispiel 36A) und 2.07 g (6.37 mmol) Cäsiumcarbonat wurden in 15 ml Aceton suspendiert und mit 1.69 g (5.52 mmol) tert-Butyl-4-(brommethyl)-3-chlorbenzoat (Herstellung gemäß WO 2003/000692, Beispiel 4b) versetzt. Es wurde 18 h bei 50°C gerührt. Man verdünnte die Suspension mit Wasser. Es wurde zweimal mit je 15 ml Ethylacetat extrahiert. Die vereinigten organischen Phasen wurden über Natriumsulfat getrocknet und am Rotationsverdampfer vom Lösungsmittel befreit. Nach Reinigung durch Flash-Chromatographie über Kieselgel durch Elution mit Cyclohexan/ Ethylacetat (Gradient 9:1 → 3:2) erhielt man 1.41 g (53 % d. Th.) der Zielverbindung in einer Reinheit von 74%

LC-MS [Methode 7] Rₜ = 2.98 min; MS [ESIpos]: m/z = 460 (M+H)⁺

¹H-NMR (400MHz, CDCl₃): δ = 0.75 - 0.83 (m, 2H), 1.00 - 1.09 (m, 2H), 1.57 (s, 9H), 3.01 (spt, 1H), 5.16 (s, 2H), 7.24 - 7.28 (m, 1H), 7.41 - 7.47 (m, 2H), 7.65 - 7.71 (m, 2H), 7.84 (dd, 1H), 7.98 (d, 1H).

### Beispiel 27A

### 3-Chlor-4-{[3-(4-chlorphenyl)-4-cyclopropyl-5-oxo-4,5-dihydro-1H-1,2,4-triazol-1-yl]methyl}benzoesäure

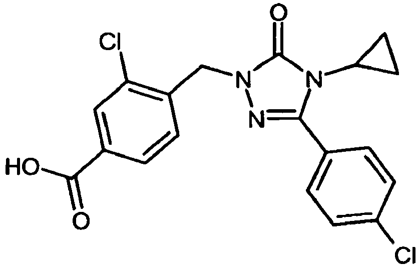

1,38 g (3.00 mmol) der Verbindung aus Beispiel 26A wurden in 10 ml Dichlormethan gelöst und bei RT mit 3.0 ml (39 mmol) Trifluoressigsäure versetzt und 72 h bei RT gerührt. Das Gemisch wurde unter vermindertem Druck eingeengt und mit 10 ml Toluol versetzt und erneut eingeengt. Diese Prozedur wurde noch weitere 2-mal wiederholt. Der verbliebene Rückstand wurde am Hochvakuum von Lösemittelresten befreit. Es wurden 1.27 g (63% d.Th.) der Zielverbindung mit einer Reinheit von 60% erhalten.

LC-MS [Methode 2] Rₜ = 1.85 min; MS [ESIpos]: m/z = 404 (M+H)⁺

### Beispiel 28A

### 4-{[3-(4-Chlorphenyl)-5-oxo-4-(3,3,3-trifluor-2-hydroxypropyl)-4,5-dihydro-1H-1,2,4-triazol-1-yl]methyl}benzoesäure

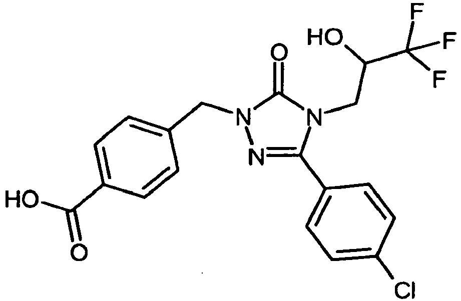

280 mg (0.61 mmol) der Verbindung aus Beispiel 24A wurde in 4 ml einer 1:1 Mischung aus Methanol und THF gelöst und mit 0.61 ml 2N Natronlauge versetzt. Es wurde 1h bei 80°C gerührt. Zur Aufarbeitung wurde mit 5 ml Wasser versetzt und mit 5 ml Ethylacetat extrahiert. Die wässrige Phase wurde mit 1N Salzsäure auf pH1 angesäuert und zweimal mit je 5 ml Ethylacetat extrahiert. Die vereinigten organischen Phasen wurden über Natriumsulfat getrocknet und am Rotationsverdampfer vom Lösungsmittel befreit. Man erhielt 275 mg (100 % d. Th.) der Zielverbindung.

LC-MS [Methode 7] Rₜ = 2.14 min; MS [ESIpos]: m/z = 442 (M+H)⁺

¹H-NMR (400MHz, DMSO-d₆): δ = 3.84 (dd, 1H), 3.96 - 4.03 (m, 1H), 4.24 - 4.36 (m, 1H), 5.08 (s, 2H), 7.42 (d, 2H), 7.60 - 7.64 (m, 2H), 7.75 (d, 2H), 7.93 (d, 2H).

### Beispiel 29A

### 3-{[3-(4-Chlorphenyl)-5-oxo-4-(3,3,3-trifluor-2-hydroxypropyl)-4,5-dihydro-1H-1,2,4-triazol-1-yl]methyl} benzoesäure

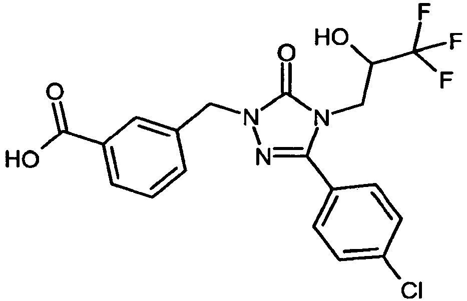

Analog zur Verbindung aus Beispiel 28A wurden 265 mg (0.58 mmol) der Verbindung aus Beispiel 25A umgesetzt. Es wurden 250 mg (97% d.Th) der Zielverbindung erhalten.

LC-MS [Methode 7] Rₜ = 2.41 min; MS [ESIpos]: m/z = 456 (M+H)⁺

¹H-NMR (400MHz, DMSO-d₆): δ = 3.84 (dd, 1H), 4.00 (dd, 1H), 4.23 - 4.34 (m, 1H), 5.00 - 5.13 (m, 2H), 7.46 - 7.53 (m, 1H), 7.54 - 7.59 (m, 1H), 7.60 - 7.64 (m, 2H), 7.74 (d, 2H), 7.88 (d, 1H), 7.94 (s, 1H).

### Ausführungsbeispiele:

### Beispiel 1

### N-tert.-Butyl-4-{[3-(4-chlorphenyl)-5-oxo-4-prop-2-en-1-yl-4,5-dihydro-1H-1,2,4-triazol-1-yl]sulfonyl}-3-methoxybenzolcarboxamid

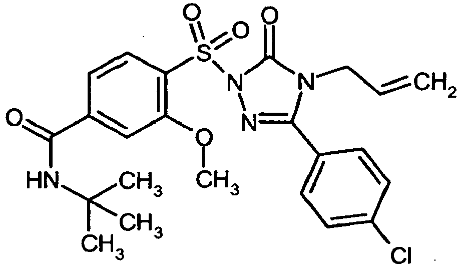

231 mg (0.24 mmol) der Verbindung aus Beispiel 3A wurden in 4 ml Tetrahydrofuran suspendiert und auf -78°C gekühlt. Zu dieser Suspension wurde unter Rühren 110 mg (0.981 mmol) Kaliumtert.-butylat gegeben. Man ließ das Gemisch auf Raumtemperatur erwärmen, der Feststoff ging dabei in Lösung. Die Reaktionslösung wurde 10 Minuten bei Raumtemperatur nachgerührt. Anschließend wurde wieder auf -78°C abgekühlt. Man gab 300 mg (0.981 mmol) 4-(tert-Butylcarbamoyl)-2-methoxybenzolsulfonylchlorid (Darstellung gemäß Liotta et. al., J.Org. Chem., 2001, 66, 3653-3661) hinzu, entfernte das Kältebad und ließ über Nacht bei Raumtemperatur weiter rühren. Man versetzte den Rückstand mit Wasser. Es wurde zweimal mit Ethylacetat extrahiert. Die vereinigten organischen Phasen wurden über Natriumsulfat getrocknet und am Rotationsverdampfer vom Lösungsmittel befreit. Der Rückstand wurde durch präparative HPLC (Methode 6) gereinigt. Man erhielt so 275 mg (55% d. Th.) der Zielverbindung

LC/MS [Methode 4]: Rₜ = 1.29 min; MS [ESIpos]: m/z = 505 (M+H)⁺

¹H-NMR (400 MHz, CDCl₃): δ = 1.47 (s, 9H), 3.94 (s, 3H), 4.27 - 4.31 (m, 2H), 5.07 (d, 1H), 5.27 (d, 1H), 5.77- 5.89 (m, 1H), 5.99 (s, NH), 7.22- 7.28 (m, 1H), 7.41- 7.46 (m, 2H), 7.49- 7.55 (m, 3H), 8.16 (d, 1H).

In analoger Weise wurden die folgenden Verbindungen erhalten (die Beispiele 3-11 sind Referenzbeispiele):

| **Beispiel Nr.** | **Struktur** | **Edukt; Ausbeute [% d.Th.]** | **¹H-NMR (400 MHz, CDCl₃)** |
|---|---|---|---|
| | | | **LC/MS: Rₜ [Methode]** |
| **2** | 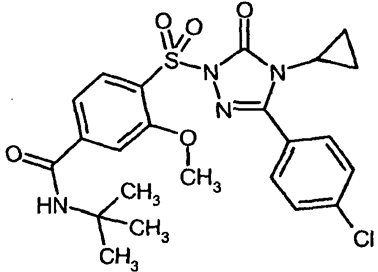 | 4A; 49% | δ = 0.69- 0.75 (m, 2H), 0.97-1.03 (m, 2H), 1.47 (s, 9H), 2.90 (m, 1H), 3.93 (s, 3H), 5.98 (br. s, 1H), 7.22- 7.26 (m, 1H), 7.46 (d, 2H), 7.51 (s, 1H), 7.68 (d, 2H), 8.16 (d, 1H). |
| | | | LC-MS [3]: Rₜ = 2.47 min; MS [ESIpos]: m/z = 505 (M+H)⁺ |
| **3** | 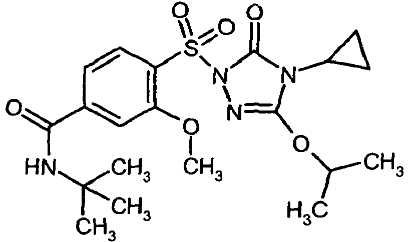 | 5A; 78% | δ = 0.91- 0.99 (m, 4H), 1.31 (s, 3H), 1.32 (s, 3H), 1.48 (s, 9H), 2.58- 2.64 (m, 1H), 3.93 *(s,* 3H), 4.95- 5.05 (m, 1H), 6.01 (br. s, 1H), 7.22 (dd, 1H), 7.50 (s, 1H), 8.08 (d, 1H). |
| | | | LC-MS [3]: R_{t;} = 2.26 min; MS [ESIpos]: m/z = 453 (M+H)⁺ |
| **4** | 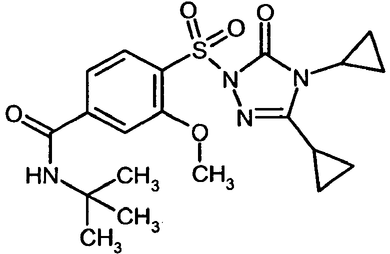 | 6A; 53% | δ = 0.98- 1.11 (m, 8H), 1.47 (s, 9H), 1.81- 1.90 (m, 1H), 2.71- 2.78 (m, 1H), 3.91 (s, 3H), 6.01 (br. s, 1H), 7.22 (dd, 1H), 7.48 (s, 1H), 8.08 (d, 1H). |
| | | | LC-MS [3]: Rₜ = 2.08 min; MS [ESIpos]: m/z = 435 (M+H)⁺ |
| **5** | 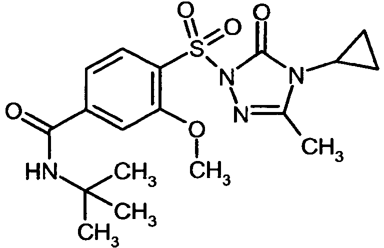 | 7A; 57% | δ = 0.94- 1.00 (m, 2H), 1.00-1.09 (m, 2H), 1.47 (s, 9H), 2.29 (s, 3H), 2.63- 2.70 (m,1H), 3.93 (s, 3H), 5.99 (br. s, 1H), 7.23 (d, 1H), 7.50 (s, 1H), 8.12 (d, 1H). |
| | | | LC-MS [5]: Rₜ = 0.97 min; MS [ESIpos]: m/z = 409 (M+H)⁺ |
| **6** | 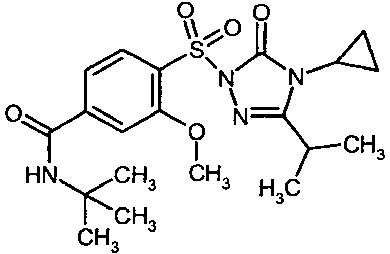 | 8A; 66% | δ = 0.96- 1.09 (m, 4H), 1.30 (d, 6H), 1.47 (s, 9H), 2.60-2.68 (m, 1H), 2.99- 3.11 (m, 1H), 3.91 (s, 3H), 6.00 (br. s, 1H), 7.23 (d, 1H), 7.49 (s, 1H), 8.12 (d, 1H). |
| | | | LC-MS [5]: Rₜ = 1.12 min; MS [ESIpos]: m/z = 437 (M+H)⁺ |
| **7** | 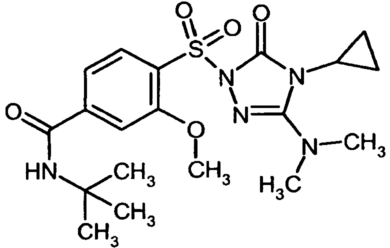 | 9A; 67% | δ = 0.95- 1.02 (m, 2H), 1.02-1.11 (m, 2H), 1.48 (s, 9H), 2.67- 2.74 (m, 1H), 2.85 (s, 6H), 3.93 (s, 3H), 6.04 (br. s, 1H), 7.23 (dd, 1H), 7.49 (d, 1H), 8.07 (d, 1H). |
| | | | LC-MS [2]: Rₜ = 1.56 min; MS [ESIpos]: m/z = 438 (M+H)⁺ |
| **8** | 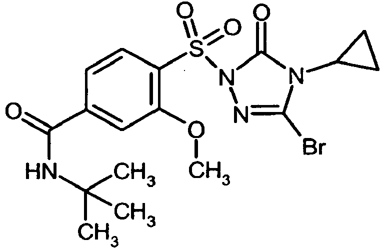 | 10A; 80% | δ = 1.01- 1.14 (m, 4H), 1.48 (s, 9H), 2.67- 2.74 (m, 1H), 3.95 (s, 3H), 5.98 (br. s, 1H), 7.24 (dd, 1H), 7.52 (d, 1H), 8.11 (d, 1H). |
| | | | LC-MS [2]: Rₜ = 1.71 min; MS [ESIpos]: m/z = 473 (M+H)⁺ |
| **9** | 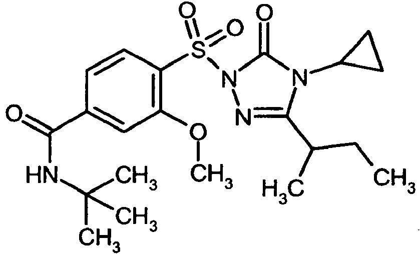 | 11A; 68% | δ = 0.88 (t, 3H), 0.94- 1.09 (m, 4H), 1.27 (d, 3H), 1.47 (s, 9H), 1.54- 1.67 (m, 1H), 1.75- 1.88 (m, 1H), 2.58-2.65 (m, 1H), 2.84- 2.94 (m, 1H), 3.90 (s, 3H), 6.00 (br. s, 1H), 7.23 (dd, 1H), 7.49 (d, 1H), 8.12 (d, 1H). |
| | | | LC-MS [3]: Rₜ = 2.28 min; MS [ESIpos]: m/z = 251 (M+H)⁺ |
| **10** | 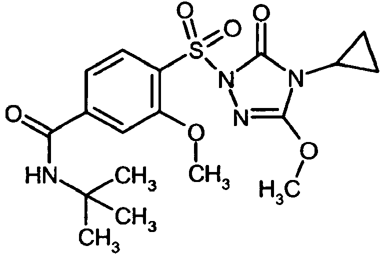 | 12A; 73% | δ = 0.92- 1.02 (m, 4H), 1.48 (s, 9H), 2.59- 2.66 (m, 1H), 3.94 (s, 3H), 3.95 (s, 3H), 6.01 (br. s, 1H), 7.23 (dd, 1H), 7.50 (d, 1H), 8.08 (d, 1H). |
| | | | LC-MS [3]: Rₜ = 2.01 min; MS [ESIpos]: m/z = 425 (M+H)⁺ |
| **11** | 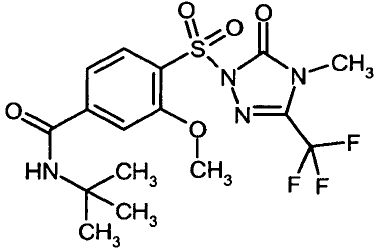 | 13A; 55% | δ = 1.48 (s, 9H), 3.35 (s, 3H), 3.94 (s, 3H), 5.98 (br. s, 1H), 7.26 (dd, 1H), 7.53 (d, 1H), 8.15 (d, 1H). |
| | | | LC-MS [4]: Rₜ = 1.14 min; MS [ESIpos]: m/z = 437 (M+H)⁺ |
| **12** | 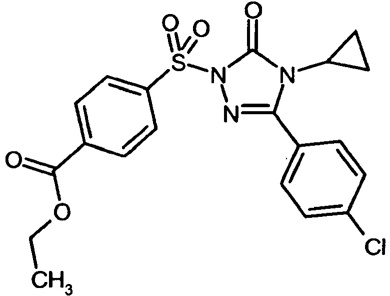 | 4A; 19% | δ = 0.68- 0.76 (m, 2H), 0.95-1.03 (m, 2H), 1.40 (t, 3H), 2.82- 2.89 (m, 1H), 4.42 (q, 2H), 7.46 (d, 2H), 7.68 (d, 2H), 8.19- 8.25 (m, 4H). |
| | | | LC-MS [3]: Rₜ = 2.70 min; MS [ESIpos]: m/z = 448 (M+H)⁺ |
| **13** | 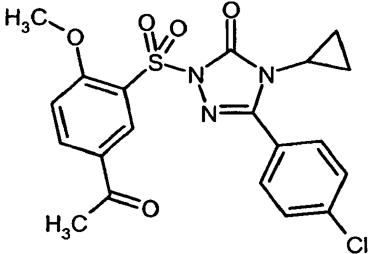 | 4A; 16% | δ = 0.71- 0.78 (m, 2H), 0.98-1.06 (m, 2H), 2.63 (s, 3H), 2.87- 2.95 (m, 1H), 3.97 (s, 3H), 7.08 (d, 1H), 7.46 (d, 2H), 7.70 (d, 2H), 8.28 (dd, 1H), 8.28 (d, 1H). |
| | | | LC-MS [3]: Rₜ = 2.30 min; MS [ESIpos]: m/z = 448 (M+H)⁺ |

### Beispiel 14

### N-tert.-Butyl-4-{[3-(4-chlorphenyl)-5-oxo-4,5-dihydro-1H-1,2,4-triazol-1-yl]sulfonyl}-3-methoxybenzolcarboxamid

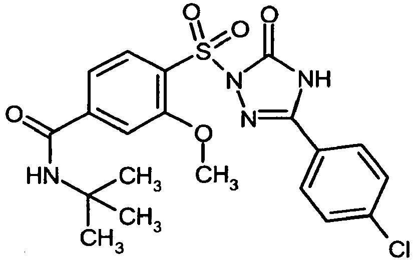

194 mg (0.384 mmol) N-tert.-Butyl-4-{[3-(4-chlorphenyl)-5-oxo-4-prop-2-en-1-yl-4,5-dihydro-1H-1,2,4-triazol-1-yl]sulfonyl}-3-methoxybenzolcarboxamid aus Beispiel 1 wurde unter Argon in 2 ml entgastem Dioxan gelöst und mit 18 mg (0.015 mmol) Tetrakis(triphenylphosphin)palladium(0), 29 µl (0.77 mmol) Ameisensäure sowie 134 µl (0.96 mmol) Triethylamin versetzt. Es wurde über 2 h unter Rückfluß gerührt. Das Dioxan wurde am Rotationsverdampfer eingeengt und der Kolbenrückstand in Methanol aufgenommen. Man filtrierte über Kieselgur, wusch mit Methanol nach und entfernte das Solvens am Rotationsverdampfer. Man versetzte den Kolbenrückstand mit Ethylacetat. Es wurde zweimal mit Wasser gewaschen. Die organische Phase wurde über Natriumsulfat getrocknet und am Rotationsverdampfer vom Lösungsmittel befreit. Nach Reinigung des Rückstands durch Flash-Chromatographie an Kieselgel (Lösemittelgradient Cyclohexan/ Ethylacetat 1:1 bis Ethylacetat 100%) erhielt man 152 mg (85% d. Th.) der Zielverbindung.

LC-MS [Methode 4] Rₜ = 1.16 min; ; MS [ESIpos]: m/z = 465 (M+H)⁺

¹H-NMR (400 MHz, CDCl₃): δ = 1.47 (s, 9H), 3.86 (s, 3H), 6.02 (s, NH), 7.24- 7.28 (m, 1H), 7.42 (d, 2H), 7.47 (s, 1H), 7.77 (d, 2H), 8.13 (d, 1H).

### Beispiel 15

### Methyl-3-{[1-{[4-(tert.-butylcarbamoyl)-2-methoxyphenyl]sulfonyl}-3-(4-chlorphenyl)-5-oxo-1,5-dihydro-4H-1,2,4-triazol-4-yl]methyl}benzolcarboxylat

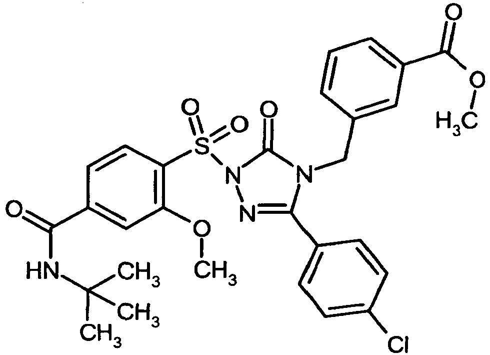

50 mg (0.108 mmol) N-tert.-Butyl-4-{[3-(4-chlorphenyl)-5-oxo-4,5-dihydro-1H-1,2,4-triazol-1-yl]sulfonyl}-3-methoxybenzolcarboxamid aus Beispiel 14 und 53 mg (0.161 mmol) Cäsiumcarbonat wurden in 1 ml Dimethylformamid suspendiert und mit 32 mg (0.140 mmol) 3-Brommethyl-benzoesäure-methylester versetzt. Es wurde über Nacht bei 60°C gerührt. Man verdünnte die Suspension mit Wasser. Es wurde zweimal mit Ethylacetat extrahiert. Die vereinigten organischen Phasen wurden über Natriumsulfat getrocknet und am Rotationsverdampfer vom Lösungsmittel befreit. Das Rohprodukt wurde durch präparative HPLC [Methode 5] gereinigt. Man erhielt so 40 mg (60% d. Th.) der Zielverbindung.

LC-MS [Methode 3] Rₜ = 2.67 min; MS [ESIpos]: m/z = 613 (M+H)⁺

¹H-NMR (400 MHz, CDCl₃): δ = 1.48 (s, 9H), 3.90 (s, 3H), 3.91 (s, 3H), 4.89 (s, 2H), 5.99 (s, NH), 7.24- 7.27 (m, 1H), 7.28- 7.34 (m, 3H), 7.38- 7.42 (m, 3H), 7.54 (d, 1H), 7.67 (s, 1H), 7.97 (d, 1H), 8.17 (d, 1H).

### Beispiel 16

### N-tert.-Butyl-4-{[3-(4-chlorphenyl)-5-oxo-4-(3,3,3-trifluor-2-hydroxypropyl)-4,5-dihydro-1H-1,2,4-triazol-1-yl]sulfonyl}-3-methoxybenzolcarboxamid

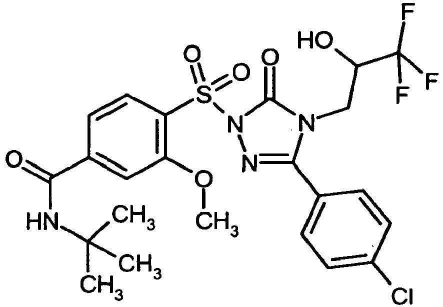

50 mg (0.108 mmol) N-tert.-Butyl-4-{[3-(4-chlorphenyl)-5-oxo-4,5-dihydro-1H-1,2,4-triazol-1-yl]sulfonyl}-3-methoxybenzolcarboxamid aus Beispiel 14 und 53 mg (0.161 mmol) Cäsiumcarbonat wurden in 1 ml Dimethylformamid suspendiert und mit 26 µl (0,161 mmol) 3-Bromo-1,1,1-trifluoro-2-propanol versetzt. Es wurde 8 Stunden bei 75°C gerührt. Man verdünnte die Suspension mit gesättigter wässriger Ammoniumchloridlösung. Es wurde zweimal mit Ethylacetat extrahiert. Die vereinigten organischen Phasen wurden über Natriumsulfat getrocknet und am Rotationsverdampfer vom Lösungsmittel befreit. Das Rohprodukt wurde durch präparative HPLC [Methode 5] gereinigt. Man erhielt so 22 mg (36% d. Th.) der Zielverbindung.

LC-MS [Methode 4] Rₜ = 1.30 min; MS [ESIpos]: m/z = 577 (M+H)⁺

¹H-NMR (400 MHz, CDCl₃): δ = 1.47 (s, 9H), 3.87- 3.97 (m, 2H), 3.96 (s, 3H), 4.40- 4.59 (s, 2H), 6.04 (s, NH), 7.21- 7.28 (m, 1H), 7.44- 7.52 (m, 3H), 7.60 (d, 2H), 8.12 (d, 1H).

### Beispiel 17

### N-tert.-Butyl-4-{[3-(4-chlorphenyl)-4-cyclopropyl-5-oxo-4,5-dihydro-1H-1,2,4-triazol-1-yl]sulfonyl}benzolcarboxamid

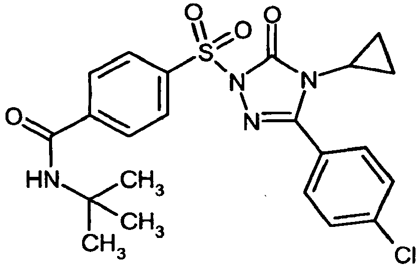

50 mg (0.113 mmol) der Verbindung aus Beispiel 15A wurden in 1 ml DMF vorgelegt und mit 18 mg (0.136 mmol) 1-Hydroxy-1H-benzotriazol-Hydrat und mit 28 mg (0.147 mmol) N'-(3-Dimethylaminopropyl)-N-ethylcarbodiimidhydrochlorid versetzt. Nach 10 min Rühren bei RT wurde 2-Methylpropan-2-amin (13.1 µl, 0.124 mmol) hinzugegeben und die Mischung über Nacht bei Raumtemperatur weitergerührt. Anschließend wurde die Reaktionslösung mit Methanol verdünnt und durch präparative HPLC [Methode 5] gereinigt. Man erhielt so 13 mg (24% d. Th.) der Zielverbindung.

LC/MS [Methode 3]: Rₜ = 2.53 min; MS [ESIpos]: m/z = 475 (M+H)⁺

¹H-NMR (400 MHz, CDCl₃): δ = 0.68 - 0.74 (m, 2 H), 0.94 - 1.02 (m, 2 H), 1.47 (s, 9 H), 2.85 (dt, 1 H), 5.93 (br. s., 1 H), 7.46 (d, 2 H), 7.67 (d, 2 H), 7.87 (d, 2 H), 8.19 (d, 2 H).

In analoger Weise wurden die folgenden Verbindungen erhalten:

| **Beispiel Nr.** | **Struktur** | **Edukt; Ausbeute [% d.Th.]** | **¹H-NMR (400 MHz, CDCl₃)** |
|---|---|---|---|
| | | | **LC/MS: Rₜ [Methode]** |
| **18** | 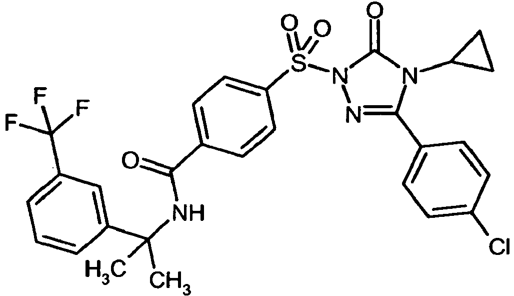 | 15A; 18% | δ = 0.67 - 0.76 (m, 2 H), 0.94 - 1.03 (m, 2 H), 1.82 (s, 6 H), 2.81 - 2.89 (m, 1 H), 6.48 (br. s., 1 H), 7.43 - 7.59 (m, 4 H), 7.59 - 7.73 (m, 4 H), 7.92 (d, 2 H), 8.23 (d, 2 H). |
| | | | LC-MS [2]: Rₜ = 2.50 min; MS [ESIpos]: m/z = 605 (M+H)⁺ |
| **19** | 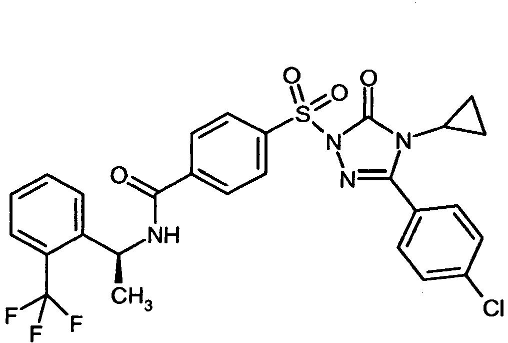 | 15A; 20% | δ = 0.66 - 0.74 (m, 2 H), 0.94 - 1.02 (m, 2 H), 1.61 (d, 3 H), 2.80 - 2.89 (m, 1 H), 5.59 (qt, 1 H), 6.52 (d, 1 H), 7.36 - 7.43 (m, 1 H), 7.46 (d, 2 H), 7.57 (d, 2 H), 7.62 - 7.75 (m, 3 H), 7.93 (d, 2 H), 8.19 (d, 2 H). |
| | | | LC-MS [3]: Rₜ = 2.75 min; MS [ESIpos]: m/z = 591 (M+H)⁺ |

### Beispiel 20

### N-tert.-Butyl-4-{[3-(4-chlorphenyl)-4-cyclopropyl-5-oxo-4,5-dihydro-1H-1,2,4-triazol-1-yl]methyl}-3-methoxybenzolcarboxamid

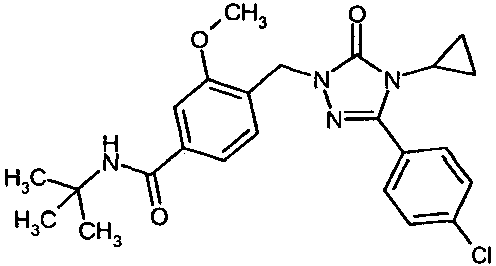

50 mg (0.13 mmol) der Verbindung aus Beispiel 21A wurden in 1 ml DMF vorgelegt und mit 20 mg (0.15 mmol) HOBt und mit 31 mg (0.16 mmol) EDC versetzt. Nach 10 min Rühren bei RT wurden 14.5µl (0.14 mmol) 2-Methylpropan-2-amin hinzugegeben und die Mischung 16 h bei Raumtemperatur weitergerührt. Anschließend wurde die Reaktionslösung mit ca. 5 ml Wasser verdünnt und der ausgefallende Niederschlag wurde filtriert und mit Wasser gewaschen. Nach dem Trocknen im Vakuum erhielt man 37 mg (66% d. Th.) der Zielverbindung.

LC-MS [Methode 2] Rₜ = 2.03 min; MS [ESIpos]: m/z = 455 (M+H)⁺

¹H-NMR (400 MHz, CDCl₃): δ = 0.74-0.80 (m, 2H), 0.99-1.06 (m, 2H), 1.46 (s, 9H), 2.96-3.03 (m, 1 H), 3.90 (s, 3H), 5.06 (s, 2H), 5.91 (br. s, 1H), 7.09 (dd, 1H), 7.16 (d, 2H), 7.38 (d, 1 H), 7.43 (d, 2H), 7.67 (d, 2H).

### Beispiel 21

### 4-{[3-(4-Chlorphenyl)-4-cyclopropyl-5-oxo-4,5-dihydro-1H-1,2,4-triazol-1-yl]methyl}-3-methoxy-N-{2-[3-(trifluormethyl)phenyl]propan-2-yl}benzamid

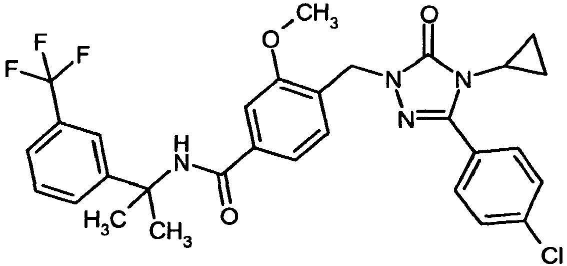

Analog zur Verbindung aus Beispiel 20 wurden aus 50 mg (0.13 mmol) der Verbindung aus Beispiel 21A 61 mg (83% d.Th.) der Zielverbindung erhalten.

LC-MS [Methode 2] Rt = 2.40 min; MS [ESIpos]: m/z = 585 (M+H)⁺

¹H-NMR (400MHz, CDCl₃): δ = 0.75-0.80 (m, 2H), 1.00-1.07 (m, 2H), 1.80 (s, 6H), 3.00 (spt, 1H), 3.88 (s, 3H), 5.07 (s, 2H), 6.43 (s, 1H), 7.17-7.23 (m, 2H), 7.37 (s, 1H), 7.41-7.51 (m, 4H), 7.61 (d, 1H), 7.63-7.69 (m, 3H).

### Beispiel 22

### N-tert.-Butyl-4-{[3-(4-chlorphenyl)-4-cyclopropyl-5-oxo-4,5-dihydro-1H-1,2,4-triazol-1-yl]methyl}-2-methoxybenzamid

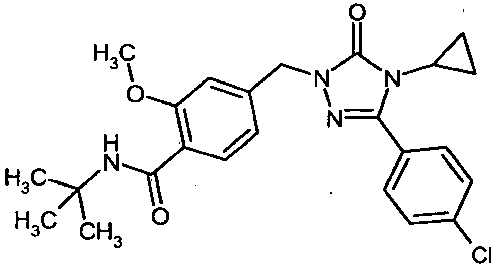

70 mg (0,30 mmol) 5-(4-Chlorphenyl)-4-cyclopropyl-2,4-dihydro-3H-1,2,4-triazol-3-on (Herstellung gemäß WO 2007/134862 Beispiel 36A) wurden in 3 ml DMF gelöst und mit 194 mg (0.59 mmol) Cäsiumcarbonat sowie 98 mg (0.33 mmol) der Verbindung aus Beispiel 23A versetzt. Es wurde 16 h bei 70 °C gerührt. Nach Abkkühlen auf RT wurde das Rohgemisch durch Filtration von unlöslichen Bestandteilen befreit und direkt chromatographisch gereinigt [Methode 8]. Man erhielt 89 mg (66% d.Th.) der Zielverbindung.

LC-MS [Methode 2] Rt = 2.12 min; MS [ESIpos]: m/z = 455 (M+H)⁺

¹H-NMR (400MHz, CDCl₃): δ = 0.72-0.78 (m, 2H), 0.99-1.05 (m, 2H), 1.44 (s, 9H), 2.94-3.01 (m, 1H), 3.95 (s, 3H), 4.98 (s, 2H), 7.03 (s, 1H), 7.09 (d, 1H), 7.44 (d, 2H), 7.65 (d, 2H), 7.76 (br. s., 1H), 8.13 (d, 1H).

### Beispiel 23

### N-tert.-Butyl-4-{[3-(4-chlorphenyl)-5-oxo-4-(3,3,3-trifluor-2-hydroxypropyl)-4,5-dihydro-1H-1,2,4-triazol-1-yl]methyl}benzamid

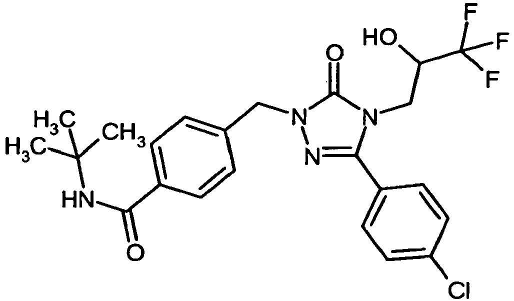

50 mg (0.11 mmol) der Verbindung aus Beispiel 28A wurden in 1 ml DMF vorgelegt und mit 18 mg (0.14 mmol) HOBt und mit 28 mg (0.15 mmol) EDC versetzt. Nach 10 min Rühren bei RT wurden 13 µl (0.12 mmol) 2-Methylpropan-2-amin hinzugegeben und die Mischung 16 h bei Raumtemperatur gerührt. Anschließend wurde die Reaktionslösung mit ca. 3 ml Wasser versetzt und zweimal mit je 5 ml Ethylacetat extrahiert. Die vereinigten organischen Phasen wurden über Natriumsulfat getrocknet, filtriert und im Vakuum eingeengt. Das Rohprodukt wurde chromatograpisch gereinigt [Methode 8]. Es wurden 31 mg (55% d. Th.) der Zielverbindung erhalten.

LC-MS [Methode 4] Rₜ = 1.29 min; MS [ESIpos]: m/z = 497 (M+H)⁺

¹H-NMR (400MHz, CDCl₃): δ = 1.46 (s, 9H), 3.95 - 4.09 (m, 2H), 4.40 - 4.50 (m, 1H), 4.98 - 5.14 (m, 3H), 5.93 (s, 1H), 7.40 (d, 2H), 7.44 - 7.53 (m, 4H), 7.65 (d, 2H).

### Beispiel 24

### 4-{[3-(4-Chlorphenyl)-5-oxo-4-(3,3,3-trifluor-2-hydroxypropyl)-4,5-dihydro-1H-1,2,4-triazol-1-yl]methyl}-N-{2-[3-(trifluormethyl)phenyl]propan-2-yl}benzamid

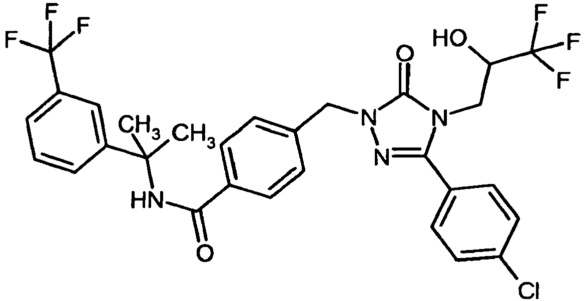

Analog zur Verbindung aus Beispiel 23 wurden aus 50 mg (0.11 mmol) der Verbindung aus Beispiel 28A 28 mg (40% d.Th.) der Zielverbindung erhalten.

LC-MS [Methode 4] Rt = 1.46 min; MS [ESIpos]: m/z = 627 (M+H)⁺

¹H-NMR (400MHz, CDCl₃): δ = 1.80 (s, 6H), 3.94 - 4.08 (m, 2H), 4.37 - 4.49 (m, 1H), 4.99 - 5.13 (m, 3H), 6.45 (s, 1H), 7.38 - 7.54 (m, 8H), 7.60 (d, 1H), 7.64 (s, 1H), 7.69 (d, 2H).

### Beispiel 25

### N-tert.-Butyl-3-{[3-(4-chlorphenyl)-5-oxo-4-(3,3,3-trifluor-2-hydroxypropyl)-4,5-dihydro-1H-1,2,4-triazol-1-yl]methyl}benzamid

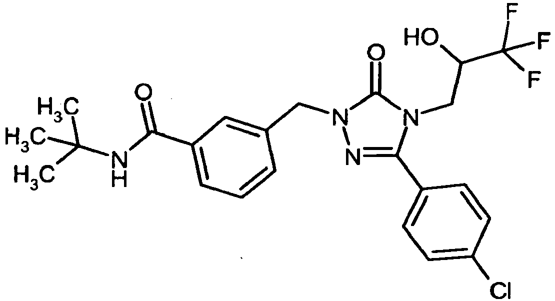

Analog zur Verbindung aus Beispiel 23 wurden aus 50 mg (0.11 mmol) der Verbindung aus Beispiel 29A 49 mg (87% d.Th.) der Zielverbindung erhalten.

LC-MS [Methode 4] Rt = 1.30 min; MS [ESIpos]: m/z = 497 (M+H)⁺

¹H-NMR (400MHz, CDCl₃): δ = 1.44 (s, 9H), 3.96 (dd, 1H), 4.06 - 4.14 (m, 1H), 4.54 - 4.65 (m, 1H), 5.00 und 5.24 (2d, 2H), 5.98 (d, 1H), 6.01 (s, 1H), 7.33 - 7.53 (m, 6H), 7.59 (d, 2H).

### Beispiel 26

### 3-{[3-(4-Chlorphenyl)-5-oxo-4-(3,3,3-trifluor-2-hydroxypropyl)-4,5-dihydro-1H-1,2,4-triazol-1-yl]methyl}-N- {2,2-difluor-2-[2-(trifluormethyl)phenyl]ethyl}benzamid

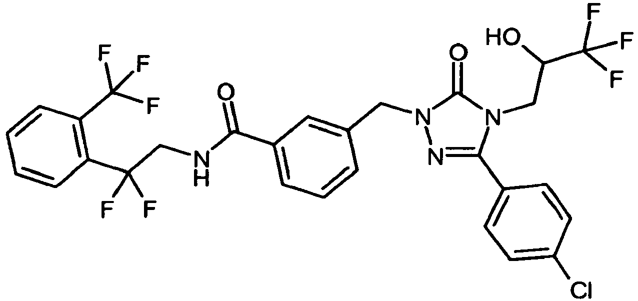

Analog zur Verbindung aus Beispiel 23 wurden aus 41 mg (0.09 mmol) der Verbindung aus Beispiel 29A 40 mg (68% d.Th.) der Zielverbindung erhalten.

LC-MS [Methode 4] Rt = 1.41 min; MS [ESIpos]: m/z = 649 (M+H)⁺

¹H-NMR (400MHz, CDCl₃): δ = 3.97 (dd, 1H), 4.04 - 4.25 (m, 3H), 4.46 - 4.60 (m, 1H), 5.02 und 5.19 (2d, 2H), 5.41 (d, 1H), 6.62 (t, 1H), 7.39 - 7.49 (m, 3H), 7.50 - 7.71 (m, 8H), 7.82 (d, 1H).

### Beispiel 27

### N-tert.-Butyl-3-chlor-4-{[3-(4-chlorphenyl)-4-cyclopropyl-5-oxo-4,5-dihydro-1H-1,2,4-triazol-1-yl]methyl} benzamid

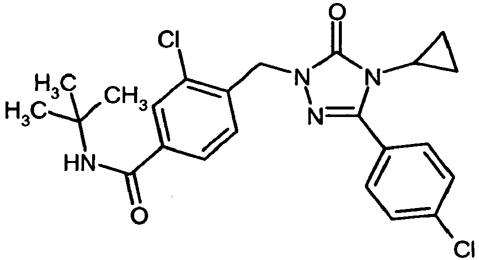

Analog zur Verbindung aus Beispiel 23 wurden aus 88 mg (ca. 0.13 mmol) der Verbindung aus Beispiel 27A 9 mg (15% d.Th.) der Zielverbindung erhalten.

LC-MS [Methode 4] Rt = 1.35 min; MS [ESIpos]: m/z = 459 und 461 (M+H)⁺

¹H-NMR (400MHz, CDCl₃): δ = 0.75 - 0.82 (m, 2H), 1.00 - 1.08 (m, 2H), 1.45 (s, 9H), 3.01 (spt, 1H), 5.15 (s, 2H), 5.85 (br. s., 1H), 7.29 (d, 1H), 7.44 (d, 2H), 7.56 (dd, 1H), 7.67 (d, 2H), 7.73 (d, 1H).

### B. Bewertung der pharmakologischen Wirksamkeit

Die pharmakologische Wirkung der erfindungsgemäßen Verbindungen kann in folgenden Assays gezeigt werden:

### Abkürzungen:

- EDTA: Ethylendiamintetraessigsäure
- DMEM: Dulbecco's Modified Eagle Medium
- FCS: Fötales Kälberserum
- HEPES: 4-(2-Hydroxyethyl)-1-piperazinethansulfonsäure
- SmGM: Smooth Muscle Cell Growth Media
- Tris-HCl: 2-Amino-2-(hydroxymethyl)-1,3-propandiol-Hydrochlorid
- UtSMC: Uterine Smooth Muscle Cells

### B-1. Zellulärer in vitro-Test zur Bestimmung der Vasopressin-Rezeptor-Aktivität

Die Identifizierung von Agonisten und Antagonisten der V1a und V2 Vasopressin Rezeptoren aus Mensch und Ratte sowie die Quantifizierung der Wirksamkeit der hier beschriebenen Substanzen erfolgte mit Hilfe von rekombinanten Zelllinien. Diese Zellen leiten sich ursprünglich von einer Ovarepithelzelle des Hamsters (Chinese Hamster Ovary, CHO K1, ATCC: American Type Culture Collection, Manassas, VA 20108, USA) ab. Die Testzelllinien expremieren konstitutiv eine modifizierte Form des Calcium-sensitiven Photoproteins Aequorin, das nach Rekonstitution mit dem Co-Faktor Coelenterazin bei Erhöhungen der freien Calcium-Konzentration im Licht emittiert (Rizzuto R., Simpson A.W., Brini M., Pozzan T.; Nature 358 (1992) 325-327). Zusätzlich sind die Zellen stabil transfiziert mit den V1a oder V2 Rezeptoren des Menschen oder der Ratte. Im Falle der Gs-koppelnden V2-Rezeptoren sind die Zellen mit einem weiteren Gen, das für das promiskuitive G_{α16}-Protein kodiert (Amatruda T.T., Steele D.A., Slepak V.Z., Simon M.I., Proc. Na. Acad. Sci. USA 88 (1991), 5587-5591), entweder unabhängig oder als Fusionsgen stabil transfiziert. Die resultierenden Vasopressin Rezeptor-Testzellen reagieren auf Stimulation der rekombinant exprimierten Vasopressin-Rezeptoren mit einer intrazellulären Freisetzung von Calcium-Ionen, die durch die resultierende Aequorin-Lumineszenz mit einem geeignetem Luminometer quantifiziert werden kann (Milligan G., Marshall F., Rees S., Trends in Pharmaco. Sci. 17 (1996) 235-237).

Testablauf: Die Zellen werden am Tag vor dem Test in Kulturmedium (DMEM, 10% FCS, 2 mM Glutamine, 10 mM HEPES) in 384-Loch-Mikrotiterplatten ausplattiert und in einem Zellinkubator (96% Luftfeuchtigkeit, 5% v/v Kohlensioxid, 37°C) gehalten. Am Testtag wird das Kulturmedium durch eine Tyrodelösung (140 mM Natriumchlorid, 5 mM Kaliumchlorid, 1 mM Magnesiumchlorid, 2 mM Calciumchlorid, 20 mM Glucose, 20 mM HEPES), das zusätzlich den Co-Faktor Coelenterazin (50 µM) enthält, ausgetauscht und die Mikrotiterplatte anschließend für weitere 3-4 Stunden inkubiert. Die Testsubstanzen werden in verschiedenen Konzentrationen für 10 bis 20 Minuten in den Löchern der Mikrotiterplatte vorgelegt, ehe der Agonist [Arg8]-Vasopressin hinzugegeben wird und das resultierende Lichtsignal sofort im Luminometer gemessen wird. Die Berechnung der IC₅₀-Werte erfolgt mit Hilfe des Computerprogramms GraphPad PRISM (Version 3.02).

In der folgenden Tabelle sind repräsentative IC₅₀-Werte für die erfindungsgemäßen Verbindungen an der mit dem humanen V1a- bzw. V2-Rezeptor transfizierten Zelllinie aufgeführt (die Beispiele 3 und 11 sind Referenzbeispiele):

**Tabelle 1:**

| **Beispiel Nr.** | **IC₅₀ hV1a [µM]** | **IC₅₀ hV2 [µM]** |
|---|---|---|
| 1 | 5.5 | 0.003 |
| 2 | 3.7 | 0.003 |
| 3 | >25 | 0.013 |
| 7 | >25 | 0.007 |
| 13 | 3.5 | 0.29 |
| 14 | > 25 | 0.009 |
| 17 | 7.1 | 0.004 |
| 20 | 15.2 | 0.004 |
| 22 | 0.87 | 0.22 |
| 27 | 4.2 | 0.027 |

### B-2. Zellulärer in vitro Test zum Nachweis der Wirkung von Vasopressin V1a Rezeptor Antagonisten auf die Regulation pro-fibrotischer Gene

Die als Kardiomyozyten-Typ beschriebene Zelllinie H9C2 (American Type Culture Collection ATCC-Nr. CRL-1446), die aus Herzgewebe der Ratte isoliert wurde, exprimiert endogen den Vasopressin V1A Rezeptor AVPR1A in hoher Kopienzahl, während die AVPR2 Expression nicht nachweisbar ist. Für Zell-Tests zur Hemmung der AVPR1A Rezeptor abhängigen Regulation der Genexpression durch Rezeptor-Antagonisten wird wie folgt verfahren:
H9C2 Zellen werden in 1.0 ml Medium Opti-MEM (Invitrogen Corp. Carlsbad CA, USA, Kat. Nr. 11058-021) mit 2% FCS und 1% Penicillin/Streptomycin Lösung (Invitrogen Kat. Nr. 10378-016) mit einer Zelldichte von 100000 Zellen/Well in 12-Well Mikrotiterplatten für die Zellkultur ausgesät und in einem Zellinkubator (96% Luftfeuchtigkeit, 5% v/v Kohlendioxid, 37°C) gehalten. Nach 24 Stunden werden in je drei Wells (Triplikate) Vehikel-Lösung (Negativ-Kontrolle), Vasopressin-Lösung [Arg8]-Vasopressin Acetat (Sigma Kat. Nr. V9879) oder Testsubstanzen (gelöst in Vehikel Wasser mit 20 Vol% Ethanol) und Vasopressin-Lösung gegeben. In der Zellkultur ist die finale Vasopressin-Konzentration 0.05 µM. Die Testsubstanz Lösung wird in kleinen Volumina zur Zellkultur gegeben, so dass eine finale Konzentration von 0.1 % Ethanol im Zelltest nicht überschritten wird. Nach einer Inkubationszeit von 6 Stunden wird der Kulturüberstand abgesaugt, die adhärenten Zellen werden in 250 µl RLT-Puffer (Qiagen, Ratingen, Kat. Nr. 79216) lysiert, und die RNA wird aus diesem Lysat mit dem RNeasy Kit (Qiagen, Kat. Nr. 74104) isoliert. Anschliessend erfolgt der DNAse-Verdau (Invitrogen Kat. Nr. 18068-015), die cDNA Synthese (Promaga ImProm-II Reverse Transcription System Kat. Nr. A3800) und die RTPCR mit dem pPCR MasterMix RT-QP2X-03-075 von Eurogentec, Seraing, Belgien. Alle Prozeduren erfolgen gemäß den Arbeitsprotokollen der Test-Reagenzien Hersteller. Die Primer-Sets für die RTPCR werden anhand der mRNA Gensequenzen (NCBI Genbank Entrez Nucleotide Data Base) mit Hilfe des Programms Primer3Plus mit 6-FAM - TAMRA markierten Sonden ausgewählt. Die RTPCR zur Bestimmung der relativen mRNA Expression in den Zellen der verschiedenen Test-Ansätze erfolgt mit dem Applied Biosystems ABI Prism 7700 Sequence Detector im 96-Well oder 384-Well Mikrotiterplatten-Format gemäß der Betriebsanleitung des Gerätes. Die relative Genexpression wird durch den delta-delta Ct-Wert dargestellt [Applied Biosystems, User Bulletin No. 2 ABI Prism 7700 SDS December 11, 1997 (updated 10/2001)] mit dem Bezug auf die Expressionsstärke des Gens Ribosomales Protein L-32 (Genbank Acc. No. NM_013226) und den Schwellen-Ct-Wert Ct = 35.

### B-3. In vivo-Test zum Nachweis der kardiovaskulären Wirkung: Blutdruckmessung an narkotisierten Ratten (Vasopressin ,Challenge' Modell)

Bei männlichen Sprague-Dawley-Ratten (250-350 g Körpergewicht) werden in Ketamin/Xylazin/Pentobarbital-Injektionsnarkose Polyethylenschläuche (PE-50; Intramedic®), die mit Heparin-haltiger (500 I.E./ml) isotoner Natriumchlorid-Lösung vorgefüllt sind, in die Vena jugularis und die Vena femoralis eingeführt und anschließend eingebunden. Über einen venösen Zugang wird mit Hilfe einer Spritze Arginin-Vasopressin injiziert, über den zweiten venösen Zugang werden die Testsubstanzen appliziert. Zur Ermittlung des systolischen Blutdruckes wird ein Druckkatheter (Millar SPR-320 2F) in die A. carotis eingebunden. Der arterielle Katheder wird an einen Druckwandler angeschlossen, der seine Signale in einen mit einer geeigneten Registrierungssoftware ausgestatteten Messcomputer einspeist. In einem typischen Experiment werden dem Versuchstier 3-4 aufeinanderfolgende Bolusinjektionen im Abstand von 10-15 min mit einer definierten Menge Arginin-Vasopressin (30 ng/kg) in isotoner Natriumchlorid-Lösung verabreicht und, nachdem der Blutdruck wieder Ausgangswerte erreicht hat, die zu testende Substanz als Bolus mit anschließender Dauerinfusion in einem geeigneten Lösungsmittel appliziert. Hiernach wird in definierten Abständen (10-15 min) erneut die gleiche Menge Vasopressin wie zu Anfang verabreicht. Anhand der Blutdruckwerte wird ermittelt, inwieweit die Testsubstanz der blutdrucksteigernden Wirkung des Vasopressins entgegenwirkt. Kontrolltiere erhalten nur Lösungsmittel statt der Testsubstanz.

Die erfindungsgemäßen Verbindungen bewirken nach intravenöser Applikation im Vergleich zu den Lösungsmittelkontrollen eine Hemmung des durch Arginin-Vasopressin verursachten Blutdruckanstiegs.

### B-4. In vivo-Test zum Nachweis der kardiovaskulären Wirkung: Diurese-Untersuchungen an wachen Ratten in Stoffwechselkäfigen

Wistar Ratten (220-400 g Körpergwicht) werden mit freiem Zugang zu Futter (Altromin) und Trinkwasser gehalten. Während des Versuches werden die Tiere für 4 bis 8 Stunden einzeln in für Ratten dieser Gewichtsklasse geeigneten Stoffwechselkäfigen (Tecniplast Deutschland GmbH, D-82383 Hohenpeißenberg) mit freiem Zugang zu Trinkwasser gehalten. Am Versuchsbeginn wird den Tieren die zu prüfende Substanz in einem Volumen von 1 bis 3 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanzdosisgruppen bestehen aus jeweils 4 bis 8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Um eine ausreichende Urinmenge zu erhalten, wird den Tieren zu Versuchsbeginn per Schlundsonde eine definierte Menge Wasser zugeführt (typischerweise 10 ml pro Kilogramm Körpergewicht). Vor Versuchsbeginn und nach Versuchsende wird das Körpergewicht der einzelnen Tiere bestimmt.

Die erfindungsgemäßen Verbindungen bewirken nach oraler Applikation im Vergleich mit Kontrolltieren eine gesteigerte Ausscheidung von Urin, die im wesentlichen auf einer gesteigerten Wasserauscheidung (Aquarese) beruht.

### B-5. In vivo-Test zum Nachweis der kardiovaskulären Wirkung: Hämodynamische Untersuchungen an narkotisierten Hunden

Männliche oder weibliche Mischlingshunde (Mongrels, Marshall BioResources, USA) mit einem Gewicht zwischen 20 und 30 kg werden mit Pentobarbital (30 mg/kg iv, Narcoren®, Merial, Deutschland) für die operativen Eingriffe und die hämodynamische und funktionellen Untersuchungstermine jeweils annarkotisiert. Alcuroniumchlorid (Alloferin®, ICN Pharmaceuticals, Deutschland, 3 mg/Tier iv) dient dabei zusätzlich als Muskelrelaxans. Die Hunde werden intubiert und mit einem Sauerstoff-Raumluft-Gemsich (40/60%) beatmet (etwa 5-6L/min). Die Beatmung erfolgt mit einem Beatmungsgerät der Firma Draeger (Sulla 808) und wird überwacht mit einem Kohlendioxid-Analysator (Engström).

Die Narkose wird aufrechterhalten durch eine ständige Infusion von Pentobarbital (50µg/kg/min); als Analgetikum dient Fentanyl (10 µg/kg/h). Eine Alternative zu Pentobarbital besteht in der Verwendung von Isofluran (1-2 Vol%).

Die Hunde werden in vorbereitenden Eingriffen mit einem Herzchrittmacher ausgestattet.
- Zum Zeitpunkt 21 Tage vor der ersten Medikamenten-Testung =(Versuchsbeginn) wird eine Herzschrittmacher der Fa. Biotronik (Logos®) in eine subcutane Hauttasche implantiert und über eine Schrittmacher-Elektrode, die durch die Vena jugularis externa unter Durchleuchtung bis in den rechten Ventrikel vorgeschoben wird, mit dem Herzen in Kontakt gebracht.
- Zum gleichen Zeitpunkt der Schrittmacher-Implantation erfolgt durch retrogrades Vorschieben eine 7F-Biopsie-Zange (Fa. Cordis) über eine Schleuse (Avanti+®; Fa. Cordis) in der Arteria femoralis und nach atraumatischer Passager der Aortenklappe eine definierte Läsion der Mitralklappe unter echokardiographischer und Durchleuchtungskontrolle. Im Anschluss werden alle Zugänge entfernt und der Hund wacht spontan aus der Narkose auf.
- Nach 7 weiteren Tagen (=14 Tage vor der ersten Medikamenten-Testung) wird der o.g. Schrittmacher aktiviert und das Herz mit einer Frequenz von 220 Schlägen pro Minute stimuliert.

Die eigentlichen Experimente zur Medikamenten-Testung erfolgen 14 und 28 Tagen nach Beginn der Schrittmacher-Stimulation nach folgender Instrumentierung:
- Blasenkatheter zur Blasenentlastung bzw. zur Messung des Urinflusses
- EKG-Ableitungen an den Extremitäten (zur EKG Messung)
- Einführen eines NaCl-gefüllten Fluidmedic-PE-300-Schlauches in die A. femoralis. Dieser ist verbunden mit einem Drucksensor (Braun Melsungen, Melsungen, Deutschland) zur Messung des systemischen Blutdrucks
- Einführen eines Millar Tip Katheters (Typ 350 PC, Millar Instruments, Houston, USA) über den linken Vorhof oder über eine in der A. carotis eingebundene Schleuse zur Messung der Herz-Hämodynamik
- Einführen eines Swan-Ganz-Katheters (CCOmbo 7.5F, Edwards, Irvine, USA) über die V. jugularis in die A. pulmonalis zur Messung von Herzzeitvolumen, Sauerstoffsättigung, Pulmonalartiendrücken und zentralvenösem Druck.
- Platzieren einer Braunüle in den Venae cephalicae zur Infusion von Pentobarbital, der Flüssigkeitssubstitution und zur Blutentnahme (Bestimmung der Substanz-Plasmaspiegeln oder sonstiger klinischer Blutwerte)
- Platzieren einer Braunüle in der Venae saphenae zur Infusion von Fentanyl und zur Substanzapplikation
- Infusion von Vasopressin (Fa. Sigma) in aufsteigender Dosierung bis zu einer Dosis von 4 mU/kg/min. Unter dieser Dosierung erfolgt dann die Testung pharmakologischer Substanzen.

Die Primärsignale werden eventuell verstärkt (Gould Verstärker, Gould Instrument Systems, Valley View, USA) bzw. Edwards-Vigilance- Monitor (Edwards, Irvine, USA) und anschließend zur Auswertung in das Ponemah-System (DataSciences Inc, Minneapolis, USA) eingespeist. Die Signale werden kontinuierlich über den gesamten Versuchszeitraum aufgezeichnet, von dieser Software digital weiterarbeitet und über 30 s gemittelt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 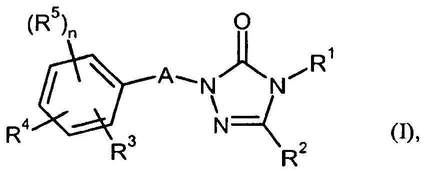 in welcher
A für -CH₂- oder -SO₂- steht,
R¹ für Wasserstoff, (C₁-C₆)-Alkyl, (C₂-C₆)-Alkenyl, (C₂-C₆)-Alkinyl oder (C₃-C₇)-Cycloalkyl steht,
wobei (C₁-C₆)-Alkyl, (C₂-C₆)-Alkenyl und (C₂-C₆)-Alkinyl mit 1 bis 3 Substituenten unabhängig voneinander ausgewählt aus der Gruppe Halogen, Cyano, Oxo, Trifluormethyl, (C₃-C₇)-Cycloalkyl, Phenyl, -OR¹⁰, -NR¹¹R¹², -C(=O)-OR¹³ und -C(=O)-NR¹⁴R¹⁵ substituiert sein können,
worin (C₃-C₇)-Cycloalkyl mit 1 oder 2 Substituenten unabhängig voneinander ausgewählt aus der Gruppe (C₁-C₄)-Alkyl, Oxo, Hydroxy, (C₁-C₄)-Alkoxy und Amino substituiert sein kann,
und
worin Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, Hydroxymethyl, (C₁-C₄)-Alkoxy, Trifluormethoxy, (C₁-C₄)-Alkoxymethyl, Hydroxycarbonyl, (C₁-C₄)-Alk-oxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹², R¹³, R¹⁴ und R¹⁵ unabhängig voneinander jeweils für Wasserstoff oder (C₁-C₆)-Alkyl stehen,
worin (C₁-C₆)-Alkyl seinerseits mit 1 oder 2 Substituenten unabhängig voneinander ausgewählt aus der Gruppe Amino, Hydroxy, (C₁-C₄)-Alkoxy, Hydroxycarbonyl und (C₁-C₄)-Alkoxycarbonyl substituiert sein kann
und
wobei (C₃-C₇)-Cycloalkyl mit 1 oder 2 Substituenten unabhängig voneinander ausgewählt aus der Gruppe (C₁-C₄)-Alkyl, (C₁-C₄)-Alkoxy, Hydroxy, Amino und Oxo substituiert sein kann,
R² für Phenyl steht,
wobei Phenyl mit 1 bis 3 Substituenten unabhängig voneinander ausgewählt aus der Gruppe Halogen, Cyano, Nitro, (C₁-C₄)-Alkyl, Trifluormethyl, Hydroxy, (C₁-C₄)-Alkoxy und Trifluormethoxy substituiert sein können,
R³ für Wasserstoff oder (C₁-C₄)-Alkoxy steht,
R⁴ für (C₁-C₆)-Alkylcarbonyl, Hydroxycarbonyl, (C₁-C₆)-Alkoxycarbonyl, Mono-(C₁-C₆)-Alkylaminocarbonyl oder Di-(C₁-C₆)-Alkylaminocarbonyl steht,
wobei (C₁-C₆)-Alkylcarbonyl, (C₁-C₆)-Alkoxycarbonyl, Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe Halogen, Trifluormethyl, (C₁-C₆)-Alkyl und Phenyl substituiert sein können,
worin Phenyl mit 1 oder 2 Substituenten unabhängig voneinander ausgewählt aus der Gruppe Halogen, Cyano, Trifluormethyl, Hydroxy-(C₁-C₄)-Alkyl, (C₁-C₄)-Alkyl, Trifluormethoxy und (C₁-C₄)-Alkoxy substituiert sein kann,
R⁵ für Halogen, Trifluormethyl, (C₁-C₄)-Alkyl, Trifluormethoxy oder (C₁-C₄)-Alkoxy steht,
n für eine Zahl 0, 1 oder 2 steht,
sowie ihre Salze, Solvate und Solvate der Salze.

2. Verbindung der Formel (I) nach Anspruch 1, in welcher
A für -CH₂- oder -SO₂- steht,
R¹ für Wasserstoff, (C₁-C₆)-Alkyl, (C₂-C₄)-Alkenyl oder (C₃-C₆)-Cycloalkyl steht,
wobei (C₁-C₆)-Alkyl und (C₂-C₄)-Alkenyl mit 1 bis 3 Substituenten unabhängig voneinander ausgewählt aus der Gruppe Fluor, Chlor, Oxo, Trifluormethyl, (C₃-C₆)-Cycloalkyl, Phenyl, -OR¹⁰, -NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, (C₁-C₄)-Alkyl, Trifluormethyl, Hydroxy, (C₁-C₄)-Alkoxy, Trifluormethoxy, Hydroxycarbonyl, (C₁-C₄)-Alkoxycarbonyl, Aminocarbonyl, Mono-(C₁-C₄)-alkylaminocarbonyl und Di-(C₁-C₄)-alkylaminocarbonyl substituiert sein kann,
und
worin R¹⁰, R¹¹, R¹², R¹³, R¹⁴ und R¹⁵ unabhängig voneinander jeweils für Wasserstoff oder (C₁-C₆)-Alkyl stehen,
R² für Phenyl steht,
wobei Phenyl mit 1 bis 3 Substituenten unabhängig voneinander ausgewählt aus der Gruppe Fluor, Chlor, Cyano, Methyl, Ethyl, Trifluormethyl, Hydroxy, Methoxy, Ethoxy und Trifluormethoxy substituiert sein kann,
R³ für Wasserstoff oder Methoxy steht,
R⁴ für (C₁-C₆)-Alkylcarbonyl, Hydroxycarbonyl, (C₁-C₆)-Alkoxycarbonyl, Mono-(C₁-C₆)-Alkylaminocarbonyl oder Di-(C₁-C₆)-Alkylaminocarbonyl steht,
wobei Mono-(C₁-C₆)-Alkylaminocarbonyl und Di-(C₁-C₆)-Alkylaminocarbonyl mit 1 bis 4 Substituenten unabhängig voneinander ausgewählt aus der Gruppe Fluor, Trifluormethyl, (C₁-C₄)-Alkyl und Phenyl substituiert sein können,
worin Phenyl mit 1 oder 2 Substituenten unabhängig voneinander ausgewählt aus der Gruppe Fluor, Chlor, Trifluormethyl, Methyl, Ethyl, Trifluormethoxy, Methoxy und Ethoxy substituiert sein kann,
R⁵ für Fluor, Chlor, Trifluormethyl, Methyl, Ethyl, Trifluormethoxy, Methoxy oder Ethoxy steht,
n für eine Zahl 0 oder 1 steht,
sowie ihre Salze, Solvate und Solvate der Salze.

3. Verbindung der Formel (I) nach Anspruch 1 oder 2, in welcher
A für -CH₂- oder -SO₂- steht,
R¹ für Wasserstoff, (C₁-C₆)-Alkyl, (C₂-C₄)-Alkenyl oder Cyclopropyl steht,
wobei (C₁-C₆)-Alkyl und (C₂-C₄)-Alkenyl mit 1 bis 3 Substituenten unabhängig voneinander ausgewählt aus der Gruppe Fluor, Oxo, Trifluormethyl, Phenyl, - OR¹⁰, -NR¹¹R¹², -C(=O)-OR¹³ und -C(=O)-NR¹⁴R¹⁵ substituiert sein können,
worin Phenyl mit 1 bis 3 Substituenten unabhängig voneinander ausgewählt aus der Gruppe Fluor, Chlor, Methyl, Ethyl, Trifluormethyl, Hydroxy, Methoxy, Ethoxy, Trifluormethoxy, Hydroxycarbonyl, Methoxycarbonyl und Ethoxycarbonyl substituiert sein kann,
und
worin R¹⁰, R¹¹, R¹², R¹³, R¹⁴ und R¹⁵ unabhängig voneinander jeweils für Wasserstoff oder Methyl stehen,
R² für Phenyl steht,
wobei Phenyl mit 1 oder 2 Substituenten unabhängig voneinander ausgewählt aus der Gruppe Fluor, Chlor, Methyl, Ethyl, Trifluormethyl, Hydroxy, Methoxy, Ethoxy und Trifluormethoxy substituiert sein kann,
R³ für Wasserstoff oder Methoxy steht,
R⁴ für Methylcarbonyl, Ethylcarbonyl, Methoxycarbonyl, Ethoxycarbonyl, Mono-(C₁-C₆)-Alkylaminocarbonyl oder Di-(C₁-C₆)-Alkylaminocarbonyl steht,
wobei Mono-(C₁-C₆)-Alkylaminocarbonyl und Di-(C₁-C₆)-Alkylamino-carbonyl mit 1 bis 4 Substituenten unabhängig voneinander ausgewählt aus der Gruppe Fluor, Trifluormethyl, Methyl, Ethyl und Phenyl substituiert sein können,
worin Phenyl mit 1 oder 2 Substituenten unabhängig voneinander ausgewählt aus der Gruppe Fluor, Chlor, Trifluormethyl, Methyl, Ethyl, Trifluormethoxy, Methoxy und Ethoxy substituiert sein kann,
R⁵ für Fluor, Chlor oder Trifluormethyl steht,
n für eine Zahl 0 oder 1 steht,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man
[A] eine Verbindung der Formel (II) 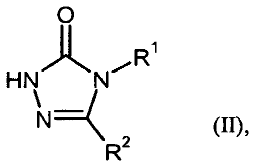 in welcher R¹ und R² jeweils die in den Ansprüchen 1 bis 3 angegebenen Bedeutungen haben,
in einem inerten Lösungsmittel in Gegenwart einer geeigneten Base mit einer Verbindung der Formel (III) 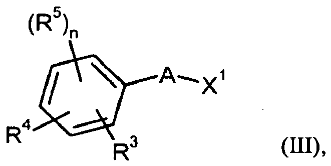 in welcher A, n, R³, R⁴ und R⁵ jeweils die in den Ansprüchen 1 bis 3 angegebenen Bedeutungen haben,
und
X¹ für Halogen, insbesondere für Chlor, steht,
umsetzt
oder
[B] von einer Verbindung der Formel (IV) 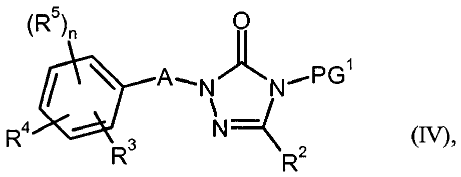 in welcher A, n, R², R³, R⁴ und R⁵ jeweils die in den Ansprüchen 1 bis 3 angegebenen Bedeutungen haben,
und
PG¹ für eine geeignete Schutzgruppe, beispielsweise Allyl, steht,
in einem inerten Lösungsmittel nach Standardmethoden die Schutzgruppe PG¹ abspaltet
und die resultierende Verbindung der Formel (V) 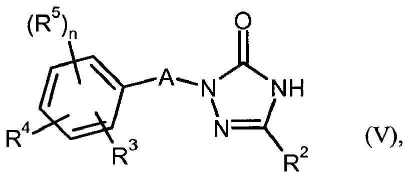 in welcher A, n, R², R³, R⁴ und R⁵ jeweils die in den Ansprüchen 1 bis 3 angegebenen Bedeutungen haben,
in Gegenwart einer geeigneten Base mit einer Verbindung der Formel (VI)
R¹-X² (VI),
in welcher R¹ die in den Ansprüchen 1 bis 3 angegebene Bedeutung hat,
und
X² für eine Abgangsgruppe, wie beispielsweise Halogen, Mesylat oder Tosylat, steht,
umsetzt
und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

5. Verbindung der Formel (I), wie in einem der Ansprüche 1 bis 3 definiert, zur Anwendung in der Behandlung und/oder Prophylaxe von Krankheiten.

6. Verbindung der Formel (I), wie in einem der Ansprüche 1 bis 3 definiert, zur Verwendung in einem Verfahren zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

7. Verwendung einer Verbindung der Formel (I), wie in einem der Ansprüche 1 bis 3 definiert, zur Herstellung eines Arzneimittels zur Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem oder mehreren weiteren Wirkstoffen ausgewählt aus der Gruppe bestehend aus Diuretika, Angiotensin AII-Antagonisten, ACE-Hemmer, beta-Rezeptoren-Blocker, Mineralocorticoid-Rezeptor-Antagonisten, organische Nitrate, NO-Donatoren und positiv-inotrop wirksame Substanzen.

10. Arzneimittel nach Anspruch 8 oder 9 zur Anwendung in der Behandlung und/oder Prophylaxe von akuter und chronischer Herzinsuffizienz, hypervolämischer und euvolämischer Hyponaträmie, Leberzirrhose, Aszites, Ödemen und des Syndroms der inadäquaten ADH-Sekretion (SIADH).

## Claims

1. Compound of the formula (I) 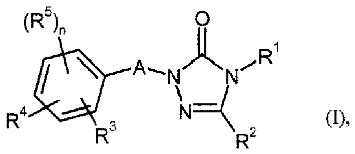 in which
A is -CH₂- or -SO₂-,
R¹ is hydrogen, (C₁-C₆) alkyl, (C₂-C₆) alkenyl, (C₂-C₆) alkynyl or (C₃-C₇) cycloalkyl,
where (C₁-C₆) alkyl, (C₂-C₆) alkenyl and (C₂-C₆) alkynyl may be substituted by 1 to 3 substituents independently of one another selected from the group consisting of halogen, cyano, oxo, trifluoromethyl, (C₃-C₇) cycloalkyl, phenyl, -OR¹⁰, -NR¹¹R¹², -C(=O)-OR¹³ and -C(=O)-NR¹⁴R¹⁵,
in which (C₃-C₇) cycloalkyl may be substituted by 1 or 2 substituents independently of one another selected from the group consisting of (C₁-C₄) alkyl, oxo, hydroxyl, (C₁-C₄) alkoxy and amino,
and
in which phenyl may be substituted by 1 to 3 substituents independently of one another selected from the group consisting of halogen, cyano, nitro, (C₁-C₄) alkyl, trifluoromethyl, hydroxyl, hydroxymethyl, (C₁-C₄) alkoxy, trifluoromethoxy, (C₁-C₄) alkoxymethyl, hydroxycarbonyl, (C₁-C₄) alkoxycarbonyl, aminocarbonyl, mono-(C₁-C₄) alkylaminocarbonyl and di-(C₁-C₄) alkylaminocarbonyl,
and
in which R¹⁰, R¹¹, R¹², R¹³, R¹⁴ and R¹⁵ are each independently of one another hydrogen or (C₁-C₆) alkyl,
in which (C₁-C₆) alkyl may on its part be substituted by 1 or 2 substituents independently of one another selected from the group consisting of amino, hydroxyl, (C₁-C₄) alkoxy, hydroxycarbonyl and (C₁-C₄) alkoxycarbonyl,
and
where (C₃-C₇) cycloalkyl may be substituted by 1 or 2 substituents independently of one another selected from the group consisting of (C₁-C₄) alkyl, (C₁-C₄) alkoxy, hydroxy, amino and oxo,
R² is phenyl
where phenyl may be substituted by 1 to 3 substituents independently of one another selected from the group consisting of halogen, cyano, nitro, (C₁-C₄) alkyl, trifluoromethyl, hydroxyl, (C₁-C₄) alkoxy and trifluoromethoxy,
R³ is hydrogen or (C₁-C₄) alkoxy,
R⁴ is (C₁-C₆) alkylcarbonyl, hydroxycarbonyl, (C₁-C₆) alkoxycarbonyl, mono-(C₁-C₆)-alkylaminocarbonyl or di-(C₁-C₆) alkylaminocarbonyl,
where (C₁-C₆)-alkylcarbonyl, (C₁-C₆) alkoxycarbonyl, mono-(C₁-C₆)-alkylaminocarbonyl and di-(C₁-C₆)-alkylaminocarbonyl may be substituted by 1 to 4 substituents independently of one another selected from the group consisting of halogen, trifluoromethyl, (C₁-C₆) alkyl and phenyl,
in which phenyl may be substituted by 1 or 2 substituents independently of one another selected from the group consisting of halogen, cyano, trifluoromethyl, hydroxyl-(C₁-C₄) alkyl, (C₁-C₄) alkyl, trifluoromethoxy and (C₁-C₄) alkoxy,
R⁵ is halogen, trifluoromethyl, (C₁-C₄) alkyl, trifluoromethoxy or (C₁-C₄) alkoxy,
n is a number 0, 1 or 2,
and also their salts, solvates, and solvates of the salts.

2. Compound of the formula (I) according to Claim 1, in which
A is -CH₂- or -SO₂-,
R¹ is hydrogen, (C₁-C₆) alkyl, (C₂-C₄) alkenyl or (C₃-C₆) cycloalkyl,
where (C₁-C₆) alkyl and (C₂-C₄) alkenyl may be substituted by 1 to 3 substituents independently of one another selected from the group consisting of fluorine, chlorine, oxo, trifluoromethyl, (C₃-C₆) cycloalkyl, phenyl, -OR¹⁰, -NR¹¹R¹², -C(=O)-OR¹³ and -C(=O)-NR¹⁴R¹⁵,
in which phenyl may be substituted by 1 to 3 substituents independently of one another selected from the group consisting of fluorine, chlorine, (C₁-C₄) alkyl, trifluoromethyl, hydroxyl, (C₁-C₄) alkoxy, trifluoromethoxy, hydroxycarbonyl, (C₁-C₄) alkoxycarbonyl, aminocarbonyl, mono-(C₁-C₄)-alkylaminocarbonyl and di-(C₁-C₄)-alkylaminocarbonyl,
and
in which R¹⁰, R¹¹, R¹², R¹³, R¹⁴ and R¹⁵ independently of one another are each hydrogen or (C₁-C₆) alkyl,
R² is phenyl,
where phenyl may be substituted by 1 to 3 substituents independently of one another selected from the group consisting of fluorine, chlorine, cyano, methyl, ethyl, trifluoromethyl, hydroxyl, methoxy, ethoxy and trifluoromethoxy,
R³ is hydrogen or methoxy,
R⁴ is (C₁-C₆) alkylcarbonyl, hydroxycarbonyl, (C₁-C₆) alkoxycarbonyl, mono-(C₁-C₆)-alkylaminocarbonyl or di-(C₁-C₆)-alkylaminocarbonyl,
where mono-(C₁-C₆)-alkylaminocarbonyl and di-(C₁-C₆)-alkylaminocarbonyl may be substituted by 1 to 4 substituents independently of one another selected from the group consisting of fluorine, trifluoromethyl, (C₁-C₄) alkyl and phenyl,
in which phenyl may be substituted by 1 or 2 substituents independently of one another selected from the group consisting of fluorine, chlorine, trifluormethyl, methyl, ethyl, trifluoromethoxy, methoxy and ethoxy,
R⁵ is fluorine, chlorine, trifluoromethyl, methyl, ethyl, trifluoromethoxy, methoxy or ethoxy,
n is a number 0 or 1,
and also their salts, solvates, and solvates of the salts.

3. Compound of the formula (I) according to Claim 1 or 2, in which
A is -CH₂- or -SO₂-,
R¹ is hydrogen, (C₁-C₆) alkyl, (C₂-C₄) alkenyl or cyclopropyl,
where (C₁-C₆) alkyl and (C₂-C₄) alkenyl may be substituted by 1 to 3 substituents independently of one another selected from the group consisting of fluorine, oxo, trifluoromethyl, phenyl, -OR¹⁰, -NR¹¹R¹², - C(=O)-OR¹³ and -C(=O)-NR¹⁴R¹⁵,
in which phenyl may be substituted by 1 to 3 substituents independently of one another selected from the group consisting of fluorine, chlorine, methyl, ethyl, trifluoromethyl, hydroxyl, methoxy, ethoxy, trifluoromethoxy, hydroxycarbonyl, methoxycarbonyl and ethoxycarbonyl,
and
in which R¹⁰, R¹¹, R¹², R¹³, R¹⁴ and R¹⁵ independently of one another are each hydrogen or methyl,
R² is phenyl,
where phenyl may be substituted by 1 or 2 substituents independently of one another selected from the group consisting of fluorine, chlorine, methyl, ethyl, trifluoromethyl, hydroxyl, methoxy, ethoxy and trifluoromethoxy,
R³ is hydrogen or methoxy,
R⁴ is methylcarbonyl, ethylcarbonyl, methoxycarbonyl, ethoxycarbonyl, mono-(C₁-C₆)-alkylaminocarbonyl or di-(C₁-C₆)-alkylaminocarbonyl,
where mono-(C₁-C₆)-alkylaminocarbonyl and di-(C₁-C₆)-alkylaminocarbonyl may be substituted by 1 to 4 substituents independently of one another selected from the group consisting of fluorine, trifluoromethyl, methyl, ethyl and phenyl,
in which phenyl may be substituted by 1 or 2 substituents independently of one another selected from the group consisting of fluorine, chlorine, trifluoromethyl, methyl, ethyl, trifluoromethoxy, methoxy and ethoxy,
R⁵ is fluorine, chlorine or trifluoromethyl,
n is a number 0 or 1, and also their salts, solvates, and solvates of the salts.

4. Process for preparing compounds of the formula (I) as defined in Claims 1 to 3, **characterized in that**
[A] a compound of the formula (II) 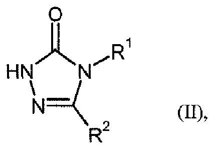 in which R¹ and R² are each as defined in Claims 1 to 3
is reacted in an inert solvent, in the presence of a suitable base, with a compound of the formula (III) 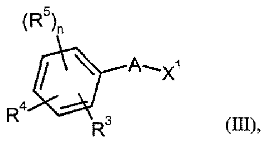 in which A, n, R³, R⁴ and R⁵ are each as defined in Claims 1 to 3, and
X¹ is halogen, in particular chlorine,
or
[B] from a compound of the formula (IV) 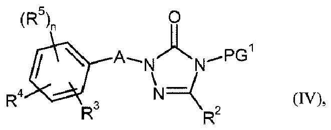 in which A, n, R², R³, R⁴ and R⁵ are each as defined in Claims 1 to 3,
and
PG¹ is a suitable protective group, for example allyl,
in an inert solvent by standard methods the protective group PG¹ is eliminated
and the resulting compound of the formula (V) 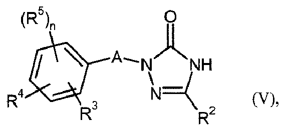 in which A, n, R², R³, R⁴ and R⁵ are each as defined in Claims 1 to 3,
is reacted in the presence of a suitable base with a compound of the formula (VI)
R¹-X² (VI),
in which R¹ is as defined in Claims 1 to 3,
and
X² is a leaving group, such as, for example, halogen, mesylate or tosylate,
and the resulting compounds of the formula (I) are converted optionally with the corresponding (i) solvents and/or (ii) bases or acids into their solvates, salts and/or solvates of the salts.

5. Compound of the formula (I) as defined in any of Claims 1 to 3 for use in the treatment and/or prophylaxis of diseases.

6. Compound of the formula (I) as defined in any of Claims 1 to 3 for use in a method for the treatment and/or prophylaxis of acute and chronic cardiac insufficiency, hypervolaemic and euvolaemic hyponatraemia, liver cirrhosis, ascites, oedemas and the syndrome of inadequate ADH secretion (SIADH).

7. Use of a compound of the formula (I) as defined in any of Claims 1 to 3 for the production of a medicament for the treatment and/or prophylaxis of acute and chronic cardiac insufficiency, hypervolaemic and euvolaemic hyponatraemia, liver cirrhosis, ascites, oedemas and the syndrome of inadequate ADH secretion (SIADH).

8. Medicament comprising a compound of the formula (I) as defined in any of Claims 1 to 3 in combination with an inert, non-toxic, pharmaceutically suitable excipient.

9. Medicament comprising a compound of the formula (I) as defined in any of Claims 1 to 3 in combination with one or more further active ingredients selected from the group consisting of diuretics, angiotensin AII antagonists, ACE inhibitors, beta receptor blockers, mineralocorticoid receptor antagonists, organic nitrates, NO donors and substances with positive inotropic activity.

10. Medicament according to Claim 8 or 9 for use in the treatment and/or prophylaxis of acute and chronic cardiac insufficiency, hypervolaemic and euvolaemic hyponatraemia, liver cirrhosis, ascites, oedemas and the syndrome of inadequate ADH secretion (SIADH).

## Revendications

1. Composé de formule (I) 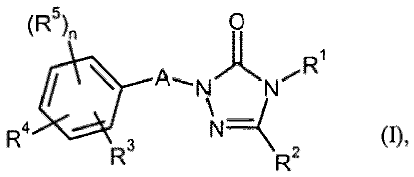 dans laquelle
A représente -CH₂- ou -SO₂-,
R¹ représente hydrogène, alkyle en (C₁-C₆), alcényle en (C₂-C₆), alcynyle en (C₂-C₆) ou cycloalkyle en (C₃-C₇),alkyle en (C₁-C₆), alcényle en (C₂-C₆) et alcynyle en (C₂-C₆) pouvant être substitués avec 1 à 3 substituants choisis indépendamment les uns des autres dans le groupe constitué par halogène, cyano, oxo, trifluorométhyle, cycloalkyle en (C₃-C₇), phényle, -OR¹⁰, -NR¹¹R¹², -C(=O)-OR¹³ et -C(=O)-NR¹⁴R¹⁵,
cycloalkyle en (C₃-C₇) pouvant être substitué avec 1 ou 2 substituants choisis indépendamment l'un de l'autre dans le groupe constitué par alkyle en (C₁-C₄), oxo, hydroxy, alcoxy en (C₁-C₄) et amino,
et
phényle pouvant être substitué avec 1 à 3 substituants choisis indépendamment les uns des autres dans le groupe constitué par halogène, cyano, nitro, alkyle en (C₁-C₄), trifluorométhyle, hydroxy, hydroxyméthyle, alcoxy en (C₁-C₄), trifluorométhoxy, alcoxyméthyle en (C₁-C₄), hydroxycarbonyle, alcoxycarbonyle en (C₁-C₄), aminocarbonyle, mono-alkylaminocarbonyle en (C₁-C₄) et di-alkylaminocarbonyle en (C₁-C₄), et
R¹⁰, R¹¹, R¹², R¹³, R¹⁴ et R¹⁵ représentant chacun indépendamment les uns des autres hydrogène ou alkyle en (C₁-C₆),
alkyle en (C₁-C₆) pouvant être substitué de son côté avec 1 ou 2 substituants choisis indépendamment l'un de l'autre dans le groupe constitué par amino, hydroxy, alcoxy en (C₁-C₄), hydroxycarbonyle et alcoxycarbonyle en (C₁-C₄),
et
cycloalkyle en (C₃-C₇) pouvant être substitué avec 1 ou 2 substituants choisis indépendamment l'un de l'autre dans le groupe constitué par alkyle en (C₁-C₄), alcoxy en (C₁-C₄), hydroxy, amino et oxo,
R² représente phényle,
phényle pouvant être substitué avec 1 à 3 substituants choisis indépendamment les uns des autres dans le groupe constitué par halogène, cyano, nitro, alkyle en (C₁-C₄), trifluorométhyle, hydroxy, alcoxy en (C₁-C₄) et trifluorométhoxy,
R³ représente hydrogène ou alcoxy en (C₁-C₄),
R⁴ représente alkylcarbonyle en (C₁-C₆), hydroxycarbonyle, alcoxycarbonyle en (C₁-C₆), mono-alkylaminocarbonyle en (C₁-C₆) ou di-alkylaminocarbonyle en (C₁-C₆),
alkylcarbonyle en (C₁-C₆), alcoxycarbonyle en (C₁-C₆), mono-alkylaminocarbonyle en (C₁-C₆) et di-alkylaminocarbonyle en (C₁-C₆) pouvant être substitués avec 1 à 4 substituants choisis indépendamment les uns des autres dans le groupe constitué par halogène, trifluorométhyle, alkyle en (C₁-C₆) et phényle,
phényle pouvant être substitué avec 1 ou 2 substituants choisis indépendamment l'un de l'autre dans le groupe constitué par halogène, cyano, trifluorométhyle, hydroxy-alkyle en (C₁-C₄), alkyle en (C₁-C₄), trifluorométhoxy et alcoxy en (C₁-C₄),
R⁵ représente halogène, trifluorométhyle, alkyle en (C₁-C₄), trifluorométhoxy ou alcoxy en (C₁-C₄),
n représente le nombre 0, 1 ou 2,
ainsi que leurs sels, solvates et solvates des sels.

2. Composé de formule (I) selon la revendication 1, dans lequel
A représente -CH₂- ou -SO₂-,
R¹ représente hydrogène, alkyle en (C₁-C₆), alcényle en (C₂-C₄) ou cycloalkyle en (C₃-C₆),
alkyle en (C₁-C₆) et alcényle en (C₂-C₄) pouvant être substitués avec 1 à 3 substituants choisis indépendamment les uns des autres dans le groupe constitué par fluor, chlore, oxo, trifluorométhyle, cycloalkyle en (C₃-C₆), phényle, -OR¹⁰, -NR¹¹R¹², -C(=O)-OR¹³ et -C(=O)-NR¹⁴R¹⁵,
phényle pouvant être substitué avec 1 à 3 substituants choisis indépendamment les uns des autres dans le groupe constitué par fluor, chlore, alkyle en (C₁-C₄), trifluorométhyle, hydroxy, alcoxy en (C₁-C₄), trifluorométhoxy, hydroxycarbonyle, alcoxycarbonyle en (C₁-C₄), aminocarbonyle, mono-alkylaminocarbonyle en (C₁-C₄) et di-alkylaminocarbonyle en (C₁-C₄),
et
R¹⁰, R¹¹, R¹², R¹³, R¹⁴ et R¹⁵ représentant chacun indépendamment les uns des autres hydrogène ou alkyle en (C₁-C₆),
R² représente phényle, phényle pouvant être substitué avec 1 à 3 substituants choisis indépendamment les uns des autres dans le groupe constitué par fluor, chlore, cyano, méthyle, éthyle, trifluorométhyle, hydroxy, méthoxy, éthoxy et trifluorométhoxy,
R³ représente hydrogène ou méthoxy,
R⁴ représente alkylcarbonyle en (C₁-C₆), hydroxycarbonyle, alcoxycarbonyle en (C₁-C₆), mono-alkylaminocarbonyle en (C₁-C₆) ou di-alkylaminocarbonyle en (C₁-C₆),
mono-alkylaminocarbonyle en (C₁-C₆) et di-alkylaminocarbonyle en (C₁-C₆) pouvant être substitués avec 1 à 4 substituants choisis indépendamment les uns des autres dans le groupe constitué par fluor, trifluorométhyle, alkyle en (C₁-C₄) et phényle,
phényle pouvant être substitué avec 1 ou 2 substituants choisis indépendamment l'un de l'autre dans le groupe constitué par fluor, chlore, trifluorométhyle, méthyle, éthyle, trifluorométhoxy, méthoxy et éthoxy,
R⁵ représente fluor, chlore, trifluorométhyle, méthyle, éthyle, trifluorométhoxy, méthoxy ou éthoxy,
n représente le nombre 0 ou 1,
ainsi que leurs sels, solvates et solvates des sels.

3. Composé de formule (I) selon la revendication 1 ou 2, dans lequel
A représente -CH₂- ou -SO₂-,
R¹ représente hydrogène, alkyle en (C₁-C₆), alcényle en (C₂-C₄) ou cyclopropyle,
alkyle en (C₁-C₆) et alcényle en (C₂-C₄) pouvant être substitués avec 1 à 3 substituants choisis indépendamment les uns des autres dans le groupe constitué par fluor, oxo, trifluorométhyle, phényle, -OR¹⁰, -NR¹¹R¹², -C(=O)-OR¹³ et -C(=O)-NR¹⁴R¹⁵,
phényle pouvant être substitué avec 1 à 3 substituants choisis indépendamment les uns des autres dans le groupe constitué par fluor, chlore, méthyle, éthyle, trifluorométhyle, hydroxy, méthoxy, éthoxy, trifluorométhoxy, hydroxycarbonyle, méthoxycarbonyle et éthoxycarbonyle, et
R¹⁰, R¹¹, R¹², R¹³, R¹⁴ et R¹⁵ représentant chacun indépendamment les uns des autres hydrogène ou méthyle,
R² représente phényle,
phényle pouvant être substitué avec 1 ou 2 substituants choisis indépendamment l'un de l'autre dans le groupe constitué par fluor, chlore, méthyle, éthyle, trifluorométhyle, hydroxy, méthoxy, éthoxy et trifluorométhoxy,
R³ représente hydrogène ou méthoxy,
R⁴ représente méthylcarbonyle, éthylcarbonyle, méthoxycarbonyle, éthoxycarbonyle, mono-alkylaminocarbonyle en (C₁-C₆) ou di-alkylaminocarbonyle en (C₁-C₆),
mono-alkylaminocarbonyle en (C₁-C₆) et di-alkylaminocarbonyle en (C₁-C₆) pouvant être substitués avec 1 à 4 substituants choisis indépendamment les uns des autres dans le groupe constitué par fluor, trifluorométhyle, méthyle, éthyle et phényle,
phényle pouvant être substitué avec 1 ou 2 substituants choisis indépendamment l'un de l'autre dans le groupe constitué par fluor, chlore, trifluorométhyle, méthyle, éthyle, trifluorométhoxy, méthoxy et éthoxy,
R⁵ représente fluor, chlore ou trifluorométhyle,
n représente le nombre 0 ou 1,
ainsi que leurs sels, solvates et solvates des sels.

4. Procédé de fabrication de composés de formule (I), tels que définis dans les revendications 1 à 3, **caractérisé en ce que**
[A] un composé de formule (II) 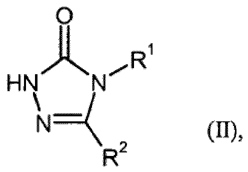 dans laquelle R¹ et R² ont chacun les significations indiquées dans les revendications 1 à 3,
est mis en réaction dans un solvant inerte en présence d'une base appropriée avec un composé de formule (III) 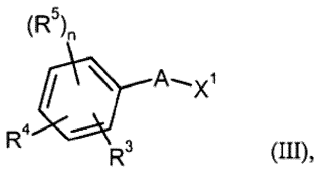 dans laquelle A, n, R³, R⁴ et R⁵ ont chacun les significations indiquées dans les revendications 1 à 3,
et
X¹ représente halogène, notamment chlore,
ou
[B] à partir d'un composé de formule (IV) 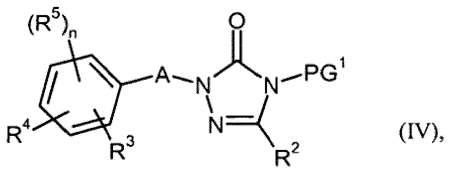 dans laquelle A, n, R², R³, R⁴ et R⁵ ont chacun les significations indiquées dans les revendications 1 à 3,
et
PG¹ représente un groupe protecteur approprié, par exemple allyle,
le groupe protecteur PG¹ est clivé dans un solvant inerte selon les méthodes standards
et le composé de formule (V) résultant 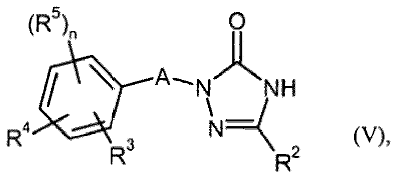 dans laquelle A, n, R², R³, R⁴ et R⁵ ont chacun les significations indiquées dans les revendications 1 à 3,
est mis en réaction en présence d'une base appropriée avec un composé de formule (VI),
R¹-X² (VI)
dans laquelle R¹ a la signification indiquée dans les revendications 1 à 3,
et
X² représente un groupe partant, tel que par exemple halogène, mésylate ou tosylate,
et les composés de formule (I) résultants sont éventuellement transformés avec les (i) solvants et/ou (ii) bases ou acides correspondants en leurs solvates, sels et/ou solvates des sels.

5. Composé de formule (I), tel que défini dans l'une quelconque des revendications 1 à 3, destiné à être utilisé pour le traitement et/ou la prophylaxie de maladies.

6. Composé de formule (I), tel que défini dans l'une quelconque des revendications 1 à 3, destiné à être utilisé dans un procédé de traitement et/ou de prophylaxie de l'insuffisance cardiaque aigue et chronique, de l'hyponatrémie hypervolémique et euvolémique, de la cirrhose du foie, de l'ascite, des oedèmes et du syndrome de sécrétion inappropriée de l'ADH (SIADH).

7. Utilisation d'un composé de formule (I), tel que défini dans l'une quelconque des revendications 1 à 3, pour la fabrication d'un médicament pour le traitement et/ou la prophylaxie de l'insuffisance cardiaque aigue et chronique, de l'hyponatrémie hypervolémique et euvolémique, de la cirrhose du foie, de l'ascite, des oedèmes et du syndrome de sécrétion inappropriée de l'ADH (SIADH).

8. Médicament contenant un composé de formule (I), tel que défini dans l'une quelconque des revendications 1 à 3, en combinaison avec un adjuvant inerte, non toxique, pharmaceutiquement approprié.

9. Médicament contenant un composé de formule (I), tel que défini dans l'une quelconque des revendications 1 à 3, en combinaison avec un ou plusieurs agents actifs supplémentaires choisis dans le groupe constitué par les diurétiques, les antagonistes de l'angiotensine AII, les inhibiteurs d'ACE, les bloquants des récepteurs bêta, les antagonistes du récepteur minéralocorticoïde, les nitrates organiques, les donneurs de NO et les substances à effet inotrope positif.

10. Médicament selon la revendication 8 ou 9, destiné à être utilisé pour le traitement et/ou la prophylaxie de l'insuffisance cardiaque aigue et chronique, de l'hyponatrémie hypervolémique et euvolémique, de la cirrhose du foie, de l'ascite, des oedèmes et du syndrome de sécrétion inappropriée de l'ADH (SIADH).
